# EUROPEAN PATENT SPECIFICATION

(11) **EP 1 481 660 B1**
(45) Date of publication and mention of the grant of the patent: **20.02.2008**
(21) Application number: 04076549.7
(22) Date of filing: 26.05.2004
(51) Int. Cl.: A61K 8/02, A61K 8/06, A61K 8/58, A61K 8/896, A61K 8/898, A61Q 1/02, A61Q 1/06, A61Q 1/08, A61Q 1/10, A61Q 1/14, A61Q 5/00, A61Q 5/06, A61Q 15/00, A61Q 17/04, A61Q 19/00, A61Q 19/10

(54) **Cosmetic**
Kosmetik
Cosmétique

(30) Priority: 26.05.2003 JP 2003147135
(43) Date of publication of application: 01.12.2004
(73) Proprietor: Shin-Etsu Chemical Co., Ltd., Chiyoda-ku, Tokyo 100-0004 (JP)
(72) Inventor: Sakuta, Koji c/o Silicone-Electric Materials Research Center Shin-Etsu Chemical Co., Ltd, Usui-gun Gunma 379-0222 (JP)
(74) Representative: Winckels, Johannes Hubertus F.

(56) References cited:
- EP-A- 1 213 006
- EP-A- 1 219 289
- EP-A- 1 241 171
- EP-A- 1 306 075
- EP-A- 1 306 077
- EP-A- 1 321 431
- EP-A- 1 369 104
- WO-A-00/13653
- WO-A-00/24364
- WO-A-99/02123
- WO-A-99/45933

## Description

This application claims benefits of Japanese Patent application No. 2003-147135 filed on May 26, 2003.

### Field of the Technology

The present invention relates to cosmetics containing a specific silicone, more particularly, a branched silicone, and having excellent feel properties as well as long lasting cosmetic coverage.

### Background of the Invention

Generally, human secretions such as sweat, tears and sebum cause makeup runs. Especially, in makeup cosmetics, an oil agent contained in cosmetics along with sebum secreted from the skin causes excessive wetting of cosmetic powder, which results in serious makeup runs. In order to reduce the amount of cosmetic oil remaining on the skin, an attempt was made to use a volatile oil such as octamethylcyclotetrasiloxane or decamethylcyclopentasiloxane as a part of the oil ingredients to be added.

Water and attrition can also be external factors causing makeup to run. To improve makeup coverage of cosmetics not to be worn off easily by sweat or tears, silicone oils with high water repellency have been added. Silicone oils such as dimethylpolysiloxane have superior properties, such as light feel to the touch, excellent water repellency, and high safety, and have recently been widely used as an oil agent in cosmetics.

However, octamethylcyclotetrasiloxane (hereinafter referred to as D4) , which has a solidification point of 17°C, has a problem that D4 crystallizes in a product in winter and causes the product to separate. In addition, when manufacturing the D4 containing products in winter, D4 must be dissolved once by heating prior to its addition, which is a source of concern in the manufacturing process. D4 is also disadvantageous since it gives dry feel to the users, which is typical of volatile cyclic silicones.

Decamethylcyclopentasiloxane (hereinafter referred to as D5), whose solidification point is -40°C, does not cause separation of the product due to crystallization in winter, but gives dry feel as D4 does.

It is known that volatile linear silicones with a viscosity of from 0.65 mm²/sec (25 degree C) to 2 mm²/sec (25 degree C) are irritant to skin and, therefore, the formulation of those silicones is not suitable for cosmetics.

From these points of view, use of tristrimethylsiloxymethylsilane (hereinafter referred to as M3T) in cosmetics is disclosed in WO01/15658. However, the cosmetics described there do not cling tightly to the skin and, therefore, there is still a need for improvement.

The inventors have found that the branched silicones with a specific structure provide cosmetics with light feel to the touch, good water repellency, and high affinity with skin as well as a long lasting coverage.

The silicones are known and, for example, Japanese Laid-Open Patent Applications Nos. 2002-256292 and 11-217584 disclose that they are effective as a cleaning agent for clothes. However, the above specifications do not describe their use as an oil agent for cosmetics or any cosmetics comprising the same. Japanese Laid-open Patent Application No. 2003-137721 (=EP-A-1306077) also discloses the use of the silicone in a rinse-off cosmetic composition. However, the rinse-off cosmetic composition is rinsed off after use and, therefore, the specification does not describe any utility of the silicone to provide cosmetics having high affinity with skin and a long lasting cosmetic coverage.

EP-A-1306075 discloses the use of the silicone in leave-on compositions for personal care.

### Disclosure of the Invention

The invention relates to compositions according to claim 1.

The branched silicone that is used in the present invention is represented by the following general formula,

*{(CH₃)₃SiO}₃SiR¹* *(1)*

wherein R¹ is a monovalent alkyl group having 2 - 20 carbon atoms and examples of R¹ include ethyl group, propyl group, butyl group, isobutyl group, pentyl group, hexyl group, octyl group, decyl group, dodecyl group, tetradecyl group, hexadecyl group, octadecyl group, etc. Preferably R¹ contains 2 - 10 carbon atoms, since, the silicone in this range has a high volatility as well as good feel to the touch. More preferably, it contains 3 - 6 carbon atoms.

Several methods are known to synthesize the silicone compounds of the present invention. For example, it can be obtained by co-hydrolysis of alkyltrichlorosilane and trimethylchlorosilane. In this method, at least 3 moles of trimethylchlorosilane is required per mole of alkyltrichlorosilane.

Japanese Laid-open Patent Application No. 2002-265478 discloses a synthesis method for low-molecular weight branched siloxanes represented by the following general formula (7), wherein trichlorosilane represented by the following general formula (5) is reacted with disiloxane represented by the following general formula (6) in the presence of a linear phosphonitrilic chloride (LPNC) catalyst:

R' SiCl₃ (5)

R₃ SiOSiR₃ (6)

R' Si (OSiR₃) (7)

wherein R is a hydrogen atom or a monovalent hydrocarbon group having 1 - 20 carbon atoms, and R' is a monovalent hydrocarbon group having 1 - 20 carbon atoms.

It can be also obtained by hydrolysis of hexamethyldisiloxane and alkyltrialkoxysilane in the presence of an acidic catalyst, wherein an acidic catalyst is added to a mixture solution of disiloxane compound represented by the following general formula (8),

R₃SiOSiR₃ (8)

wherein R is a hydrogen atom or a substituted or un-substituted monovalent hydrocarbon group having 1 to 20 carbon atoms, in alcohol, subsequently a trialkoxysilane compound represented by the following general formula (9),

R'Si(OR'')₃ (9)

wherein R' and R' ' represent a substituted or un-substituted monovalent hydrocarbon group having 1 - 20 carbon atoms, is added to react, and then water is added to perform hydrolysis to obtain a branched tetrasiloxane represented by the following general formula (10),

R'Si(OSiR₃)₃ (10)

wherein R and R' have the same meaning as above.

Examples of alkyltrialkoxysilanes represented by (9) include ethyltrimethoxysilane, ethyltriethoxysilane, ethyltripropoxysilane, propyltrimethoxysilane, propyltriethoxysilane, propyltripropoxysilane, butyltrimethoxysilane, butyltriethoxysilane, butyltripropoxysilane, hexyltrimethoxysilane, hexyltriethoxysilane, hexyltripropoxysilane, octyltrimethoxysilane, octyltriethoxysilane, octyltripropoxysilane, decyltrimethoxysilane, decyltriethoxysilane, decyltripropoxysilane, dodecyltrimethoxysilane, dodecyltriethoxysilane, and dodecyltripropoxysilane.

Preferably, the amount of the disiloxane compound that is used in the above synthesis can be more than 1.5 moles, particularly from 1.5 to 10 moles, relative to 1 mole of the trialkoxysilane. If a less amount of the disiloxane is used than the above-mentioned range, a yield of the branched tetrasiloxane will decrease and if a more amount of the disiloxane is used than the above-mentioned range, no more improvement on the yield can be attained and a pot yield may be decreased.

Examples of the alcohols used in the reaction include methanol, ethanol, *n*-propanol, isopropanol, *n*-butanol, isobutanol, and etc. Preferably, use is made of methanol, ethanol, and isopropanol. The alcohol is preferably used in an amount of from 0.5 to 5 moles, more preferaly from 1 to 3 moles, relative to 1 mole of trialkoxysilane. A yield of the branched tetrasiloxane may decrease if a less amount of the alcohol is used than the above-mentioned range and a pot yield may decrease if a more amount of the alcohol is used than the above-mentioned range.

Examples of the acidic catalyst include sulfuric acid, hydrochloric acid, methanesulfonic acid, and trifluoromethanesulfonic acid. Particularly, sulfuric acid and trifluoromethanesulfonic acid are preferred. The amount of the acid catalyst that is used in the present invention ranges preferably from 0.001 to 0.5 moles, more preferably, from 0.01 to 2 moles. If a less amount of the catalyst is used than the above-mentioned range, a reaction time will be prolonged due to a retarded reaction rate and if a more amount of the catalyst is used than the above-mentioned range, the yield may decrease since the branched tetrasiloxanes polymerize through a redistribution reaction.

The branched silicone compound thus obtained to be in the present invention is represented by the above general formula (1). Specific examples include tristrimethylsiloxyethylsilane, tristrimethylsiloxypropylsilane, tristrimethylsiloxybutylsilane, tristrimethylsiloxypentylsilane, tristrimethylsiloxyhexylsilane, tristrimethylsiloxyheptylsilane, tristrimethylsiloxyoctylsilane, tristrimethylsiloxynonylsilane, tristrimethylsiloxydecylsilane, tristrimethylsiloxydodecylsilane, tristrimethylsiloxytetradecylsilane, tristrimethylsiloxyhexadecylsilane, and tristrimethylsiloxyoctadecylsilane. Preferred are tristrimethylsiloxyethylsilane, tristrimethylsiloxypropylsilane, tristrimethylsiloxybutylsilane, tristrimethylsiloxypentylsilane, tristrimethylsiloxyhexylsilane, tristrimethylsiloxyheptylsilane, tristrimethylsiloxyoctylsilane, tristrimethylsiloxynonylsilane, and tristrimethylsiloxydecylsilane, since these vaporize quickly when applied on the skin. More preferred are tristrimethylsiloxypropylsilane, tristrimethylsiloxybutylsilane, tristrimethylsiloxypentylsilane, and tristrimethylsiloxyhexylsilane.

The cosmetic composition of the present invention comprises the silicone compound represented by the above general formula (1) . The cosmetic composition of the present invention may further comprise an oil agent (b).

The content of the volatile branched silicone compound (a) in the cosmetic composition of the present invention ranges from 1.0 mass % to 99. 0 mass %, preferably from 5.0 to 50.0 mass %.

The oil agent (b) that may be used in the present invention may be liquid, semi-solid, or solid at an ambient temperature, and preferably the oil agent (b) is liquid at an ambient temperature, and may be natural fats and oils from animals and plants, semi-synthetic oils, hydrocarbon oils, higher fatty acids, higher alcohols, ester oils, glyceride oils, silicone oils, and flouorinated oils, etc.

Examples of the natural fats and oils from animals and plants and semi-synthetic oils include avocado oil, linseed oil, almond oil, Ibota wax, perilla oil, olive oil, cacao butter, kapok wax, kaya oil, carnauba wax, Glycyrrhiza oil, candelilla wax, beef tallow, neat' s-foot oil, beef bone fat, hydrogenated beef tallow, apricot kernel oil, spermaceti wax, hydrogenated oil, wheat germ oil, sesame oil, rice germ oil, rice bran oil, sugar cane wax, sasanqua oil, safflower oil, shear butter, Chinese tung oil, cinnamon oil, jojoba wax, shellac wax, turtle oil, soybean oil, tea seed oil, camellia oil, evening primrose oil, corn oil, lard, rapeseed oil, Japanese tung oil, rice bran oil, germ oil, horse fat, persic oil, palm oil, palm kernel oil, castor oil, hydrogenated castor oil, castor oil fatty acid methyl ester, sunflower oil, grape oil, bayberry wax, jojoba oil, macadamia nut oil, beeswax, mink oil, cottonseed oil, cotton wax, Japanese wax, Japanese wax kernel oil, montan wax, coconut oil, hydrogenated coconut oil, tri-coconut oil fatty acid glyceride, mutton tallow, peanut oil, lanolin, liquid lanolin, hydrogenated lanolin, lanolin alcohol, hard lanolin, lanolin acetate, isopropyl lanolate, hexyl laurate, POE lanolin alcohol ether, POE lanolin alcohol acetate, polyethylene glycol lanolate, POE hydrogenated lanolin alcohol ether, and egg yolk oil.

Examples of the hydrocarbon oils include ozokerite, squalane, squalene, ceresin, paraffin, paraffin wax, liquid paraffin, pristane, polyisobutylene, microcrystalline wax, and Vaseline.

Examples of the higher fatty acids include lauric acid, myristic acid, palmitic acid, stearic acid, behenic acid, undecylenic acid, oleic acid, linoleic acid, linolenic acid, arachidonic acid, eicosapentaenoic acid (EPA), docosahexaenoic acid (DHA), isostearic acid, and 12-hydroxystearic acid.

Examples of the higher alcohols include lauryl alcohol, myristyl alcohol, palmityl alcohol, stearyl alcohol, behenyl alcohol, hexadecyl alcohol, oleyl alcohol, isostearyl alcohol, hexyldodecanol, octyl dodecanol, cetostearyl alcohol, 2-decyltetradecinol, cholesterol, phytosterol, sitosterol, lanosterol, POE cholesterol ether, POE phytosterol ether, monostearyl glycerin ether (batyl alcohol), and monooleyl glyceryl ether (cerakyl alcohol), etc.

Examples of the ester oils include diisobutyl adipate, 2-hexyldecyl adipate, di-2-heptylundecyl adipate, N-alkyl glycol monoisostearate, isocetyl isostearate, trimethylolpropane triisostearate, ethylene glycol di-2-ethylhexanoate, cetyl 2-ethylhexanoate, trimethylolpropane tri-2-ethylhexanoate, pentaerythritol tetra-2-ethylhexanoate, cetyl octanoate, octyldodecyl gum ester, oleyl oleate, octyldodecyl oleate, decyl oleate, neopentyl glycol dicaprirate, triethyl citrate, 2-ethylhexyl succinate, amyl acetate, ethyl acetate, butyl acetate, isocetyl stearate, butyl stearate, diisopropyl sebacinate, di-2-ethylhexyl sebacinate, cetyl lactate, myristyl lactate, isopropyl palmitate, 2-ethylhexyl palmitate, 2-hexyldecyl palmitate, 2-heptylundecyl palmitate, cholesteryl 12-hydroxystearate, dipentaerythritol fatty acid esters, isopropyl myristate, octyldodecyl myristate, 2-hexyldecyl myristate, myristyl myristate, hexyldecyl dimethyloctanoate, ethyl laurate, hexyl laurate, 2-octyldodecyl N-lauroyl-L-glutamate, and diisostearyl malate.

Examples of the glyceride oils include acetoglyceryl, glycerol triisooctanoate, glyceryl triisostearate, glyceryl triisopalmitate, glyceryl monostearate, glyceryl di-2-heptylundecanoate, glyceryl trimyristate, and di-glyceryl myristate isostearate.

Examples of the silicone oils include those represented by the following formulae,

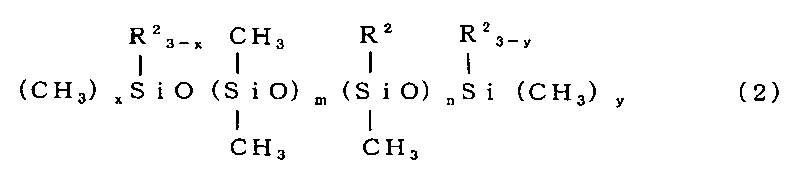

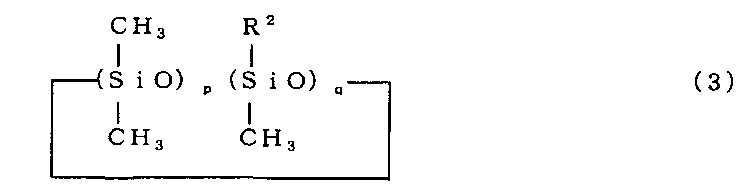

*(CH₃)₄₋ᵣSi[OSi(CH₃)₃]ᵣ (4)*

wherein R² represents a group selected from a hydrogen atom, hydroxyl group, monovalent unsubstituted or fluorine-substituted alkyl groups, aryl groups, amino-substituted alkyl groups, alkoxy groups having 2 to 20 carbon atoms, and a group represented by the general formula: (CH₃)₃SiO{(CH₃)₂SiO}₂ Si(CH₃)₂CH₂CH₂-, m is an integer of from 0 to 1,000, n is an integer of from 0 to 1,000, m + n is an integer of from 1 to 2,000, x and y are each 0,1, 2, or 3, p and q are each an integer of from 0 to 8 with 3 ≦ p + q ≦ 8, r is an integer of from 1 to 4, and s is an integer of from 0 to 500.

Preferred examples of R² include ethyl group, propyl group, butyl group, hexyl group, octyl group, decyl group, dodecyl group, tetradecyl group, hexadecyl group, octadecyl group, trifluoropropyl group, nonafluorohexyl group, heptadecylfluorodecyl group, phenyl group, aminopropyl group, dimethylaminopropyl group, aminoethylaminopropyl group, stearoxy group, butoxy group, ethoxy group, propoxy group, cetyloxy group, myristyloxy group, styryl group, α-methylstyryl group and hexyl group, octyl group, decyl group, dodecyl group, tetradecyl group, hexadecyl group,octadecyl group, trifluoropropyl group, phenyl group, aminopropyl group, aminoethylaminopropyl group.

Examples of the above-mentioned silicone oils include organopolysiloxanes which have a low to high viscosity and are liquid at ambient temperatures, such as dimethylpolysiloxane, methylphenylpolysiloxane, methylhydrogenpolysiloxane, dimethylsiloxane / methylphenylsiloxane copolymer; cyclic siloxane such as octamethylcyclotetrasiloxane (D4), decamethylcyclopentasiloxane (D5), dodecamethylcyclohexasiloxane (D6), tetramethyltetrahydrogencyclotetrasiloxane (H4), tetramethyltetraphenylcyclotetrasiloxane; branched siloxane such as tristrimethylsiloxysilane (M3T), tetrakistrimethylsiloxysilane (M4Q), tristrimethylsiloxyphenylsilane; higher alkoxy-modified silicone such as stearoxy silicone, alkyl-modified silicone, amino-modified silicone, and fluorine-modified silicone.

Examples of the fluorine-modified oils include perfluoropolyethers, perfluorodecaline, perfluorooctane, fluorinated pitch, and fluoroalcohols and one or two or more of these may be used as required.

The content of the oil agent (b) in the cosmetics of the present invention ranges from 1.0 to 99.0 mass %, preferably from 1.0 to 50.0 mass %, depending on the form of the cosmetic. When the content of (b) is less than 1.0 mass %, the effect of (b) may be lost, and when it is more than 50.0 mass %, the effect of the silicone compound (a) may be lost.

The cosmetics of the present invention may further comprise the compound having alcoholic hydroxyl group (c). The compound having alcoholic hydroxyl group (c) is preferably a water soluble mono-and/or poly-alcohol having 2 to 10 carbon atoms.

In this case, preferably the content of (c) ranges from 0.1 to 50.0 mass %, depending on the form of the cosmetic. If it is less than 0.1 mass %, the effect of (c) as moisturizer, antiseptic, and antibiotics becomes insufficient and if it is more than 50.0 mass %, stickiness increases, which is unfavorable as cosmetic.

Examples of Component (c) include lower monoalcohols such as ethanol, propanol, and isopropanol; polyalcohols such as ethylene glycol, propylene glycol, 1,3-butylene glycol, diethylene glycol, di propylene glycol polyethylene glycol, isoprenol, glycerin, diglycerin, triglycerin; and sugar alcohols such as sorbitol and maltose.

The cosmetics of the present invention may comprise one or more water-soluble or water-swelling polymer (d). Examples of the water-soluble or water-swelling polymer include plant polymers such as gum Arabic, tragacanth gum, arabinogalactan, locust bean gum (carob gum), guar gum, karaya gum, carrageenan, pectin, agar, quince seed (i.e., marmelo), starch from rice, corn, potato or wheat, algae colloid, and trant gum; bacteria-derived polymers such as xanthan gum, dextran, succinoglucan, and pullulan; animal-derived polymers such as collagen, casein, albumin, and gelatin; starch-derived polymers such as carboxymethyl starch and methylhydroxypropyl starch; cellulose polymers such as methyl cellulose, ethyl cellulose, methylhydroxypropyl cellulose, carboxymethyl cellulose, hydroxymethyl cellulose, hydroxypropyl cellulose, nitrocellulose, sodium cellulose sulfate, sodium carboxymethyl cellulose, crystalline cellulose, and cellulose powder; alginic acid-derived polymers such as sodium alginate and propylene glycol alginate; vinyl polymers such as polyvinyl methylether, and carboxyvinyl polymer; polyoxyethylene polymers; polyoxyethylene/polyoxypropylene copolymers; acrylic polymers such as sodium polyacrylate, polyethyl acrylate, and polyacrylamide; polyethyleneimine; cationic polymers; and water-soluble inorganic polymers such as, bentonite, aluminum magnesium silicate, laponite, hectorite, and silicic anhydride. Film forming agents, such as polyvinyl alcohol and polyvinyl pyrrolidone, are also included.

The content of (d) preferably ranges from 0.01 to 25.0 mass %. If it is less than 0.01 mass %, thixotropy and film forming property are insufficient and if it is more than 25.0 mass %, stickiness increases, which is unfavorable as cosmetic.

The present cosmetic composition may comprise water (e) as a constituent component and the content of (e) may range from 1. 0 to 90.0 mass %, depending on the form of the cosmetic. If it is less than 1.0 mass %, the cosmetic does not provide hydrated feel, and if it is more than 90.0 mass %, the affinity with skin decreases, which is unfavorable.

The cosmetic compositions of the present invention can be obtained by using the above-described constituent ingredients from (a) to (e) and can also comprise the following components as required; powder and coloring agents(f), surfactants (g), cross-linked organopolysiloxanes (h), silicone resins which are gummy or solid at an ambient temperatures (i), and UV protecting agents (j).

Any powder and coloring agents which are commonly used in cosmetics may be used in the present invention, regardless of the shape (spherical, rod-like, acicular, tubular, irregular, scaly or spindle forms), particle size (size of fume, fine particles or pigment grade), and particle structure (porous and non-porous), such as, for example, inorganic powder, organic powder, surface activating metal salt powder, colored pigments, pearl pigments, metallic powder pigments, and natural dyes.

Specific examples of the inorganic powders include titanium oxide, zirconium oxide, zinc oxide, cerium oxide, magnesium oxide, barium sulfate, calcium sulfate, magnesium sulfate, calcium carbonate, magnesium carbonate, talc, mica, kaolin, sericite, white mica, synthetic mica, phlogopite, lepidolite, biotite, lithia mica, silicic acid, silicic anhydride, aluminum silicate, magnesium silicate, aluminum magnesium silicate, calcium silicate, barium silicate, strontium silicate, metal salts of tungstenic acid, hydroxyapatite, vermiculite, higilite, bentonite, montmorillonite, hectolitre, zeolite, ceramics powder, calcium secondary phosphate, alumina, aluminum hydroxide, boron nitride, and silica.

Examples of the organic powders include polyamide powder, polyester powder, polyethylene powder, polypropylene powder, polystyrene powder, polyurethane, benzoguanamine powder, polymethylbenzoguanamine powder, tetrafluoroethylene powder, polymethylmethacrylate powder, cellulose, silk powder, nylon powder such as Nylon 12 and Nylon 6, spherical silicone elastomer powder having cross-linked dimethylsilicone structure (see Japanese Laid-Open Patent Application No. 3-93834), spherical polymethylsilsesquioxane powder(see Japanese Laid-Open Patent Application No. 3-47848), spherical silicone elastomer powder with its surface coated with polymethylsilsesquioxane (see Japanese Laid-Open Patent Application No. 7-196815) styrene/acrylic acid copolymer, divinylbenzene/styrene copolymer, vinyl resin, urea resin, phenol resin, fluororesin, acrylic resin, melamine resin, epoxy resin, polycarbonate resin, microcrystalline fiber powder, starch powder, and lauroyl lysine.

Examples of the surface activating metal salt powders (metal soaps) include zinc stearate, aluminum stearate, calcium stearate, magnesium stearate, zinc myristate, magnesium myristate, zinc cetyl phosphate, calcium cetyl phosphate, and zinc/sodium cetyl phosphate.

Examples of the colored pigments include inorganic red pigments such as iron oxide, iron hydroxide, and iron titanate, inorganic brown pigments such as γ-iron oxide, inorganic yellow pigments such as iron oxide yellow and loess, inorganic black pigments such as iron oxide black and carbon black, inorganic violet pigments such as manganese violet and cobalt violet, inorganic green pigments such as chromium hydroxide, chromium oxide, cobalt oxide, and cobalt titanate, inorganic blue pigments such as Prussian blue and ultramarine blue, lakes of tar pigments, lakes of natural dyes, and synthetic resin powder complexes thereof.

Examples of the pearl pigments include titanium oxide-coated mica, bismuth oxychloride, titanium oxide-coated bismuth oxychloride, titanium oxide-coated talc, fish scales, and titanium oxide-coated colored mica and examples of metallic powder pigments include aluminum powder, copper powder and stainless steel powder.

Examples of the tar pigments include Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 505, Yellow No. 4, Yellow No. 5, Yellow No. 202, Yellow No. 203, Yellow No. 204, Yellow No. 401, Blue No. 1, Blue No. 2, Blue No. 201, Blue No. 404, Green No. 3, Green No. 201, Green No. 204, Green No. 205, Orange No. 201, Orange No. 203, Orange No. 204, Orange No. 206, and Orange No. 207. Examples of the natural dyes include carminic acid, laccaic acid, carthamin, brazilin, and crocin.

These powders can be used in the form of composite or can be subject to treatment with common oil agents, silicone oils, fluorine-containing compounds or surfactants prior to use as far as the effect of the present invention is not prevented. For example, these powders may be or may not be surface-treated or modified in advance for example, by treatment with a fluorine-containing compound, treatment with a silicone resin, pendant treatment, treatment with a silane coupling agent, treatment with a titanium coupling agent, treatment with an oil agent, treatment with N-acylated lysine, treatment with a polyacrylic acid, treatment with a metal soap, treatment with an amino acid, treatment with an inorganic compound, plasma treatment, and mechanochemical treatment. If necessary, one or more kinds of surface treatment or modification can be applied.

Among these powders, preferred are spherical silicone elastomer powder, polyethylene powder, polypropylene powder, polytetrafluoroethylene powder, spherical polymethylsilsesquioxane powder, spherical silicone elastomer powder with its surface coated with polymethylsilsesquioxane, and polyurethane powder, since they can improve the stability with time and feeling touch of the product.

The content of the powder and/or coloring agent (f) to be blended is approximately from 0.1 to 50 mass%, preferably from 0.5 to 30 mass%, relative to the total amount of the cosmetic, which significantly depends upon the form of the cosmetic.

The surfactant (g) may be anionic, cationic, non-ionic or amphoteric. There is not any particular limitation and any surfactant that can be used in common cosmetics may be used.

Specific examples of the anionic surfactants include fatty acid soaps, such as sodium stearate and triethanolamine palmitate, alkylether carboxylic acids and salts thereof, salts of amino acids and fatty acids, alkylsulfonic acids, alkenesulfonates, sulfonates of fatty acid esters, sulfonates of fatty acid amides, sulfonates of alkylsulfonate-formalin condensates, alkylsulfates, sulfates of secondary higher alcohols, alkyl/allyl ether sulfates, sulfates of fatty acid esters, sulfates of fatty acid alkylolamides, and sulfates of Turkey Red oil, alkyl phosphates, ether phosphates, alkylallylether phosphates, amide phosphates, and N-acylamino surfactants.

Examples of the cationic surfactants include amine salts such as salts of alkylamine, polyamine and amino alcohol fatty acid derivatives, alkyl quaternary ammonium salts, aromatic quaternary ammonium salts, pyridium salts and imidazolium salts.

Examples of the nonionic surfactants include sorbitan fatty acid esters, glycerin fatty acid esters, polyglycerin fatty acid esters, propylene glycol fatty acid esters, polyethylene glycol fatty acid esters, sucrose fatty acid esters, polyoxyethylene alkylethers, polyoxypropylene alkylethers, polyoxyethylene alkylphenylether, polyoxyethylene fatty acid esters, polyoxyethylene sorbitan fatty acid esters, polyoxyethylene sorbitol fatty acid esters, polyoxyethylene glycerin fatty acid esters, polyoxyethylene propylene glycol fatty acid esters, polyoxyethylene castor oil, polyoxyethylene hydrogenated castor oil, polyoxyethylene phytostanolether, polyoxyethylene phytosterolether, polyoxyethylene cholestanolether, polyoxyethylene cholesterylether, polyoxyalkylene-modifed organopolysiloxane (see Japanese Patent No. 2137062 and Japanese Laid-open Patent Application No. 7-330907), polyglycerin-modified organopolysiloxane (Japanese Laid-open Patent Application No. 62-34039, and Japanese Patent Application Nos. 2613124, 2844453, and 2002-179798), polyoxyalkylene / alkyl co-modified-organopolysiloxane (Japanese Laid-open Patent Nos. 61-90732 and 9-59386) alknolamide, sugar ethers, and sugar amides.

Examples of the amphoteric surfactants include betaine, aminocarboxylates, and imidazoline derivatives. A desirable amount of the surfactant to be added ranges from 0.1 to 20 mass%, preferably from 0.5 to 10 mass%, relative to the total amount of the cosmetic.

It is preferred that the cross-linked organopolysiloxane (h) absorbs an oil agent, in particular silicones with viscosities of from 0.65 to 100.0 mm² / sec, in a larger amount by weight than the amount of the cross-linked organopolysiloxane itself to swell. This cross-linked organopolysiloxane can be obtained by reacting alkylhydrogen polysiloxane with cross-linking agent having reactive vinylic unsaturated group at molecular terminal. Examples of the alkylhydrogenpolysiloxanes include linear or partially branched methylhydrogenpolysiloxane, methylhydrogenpolysiloxane grafted with alkyl chains having 6 - 20 carbon atoms, methylhydrogenpolysiloxane grafted with polyoxyethylene chain. On average, two or more hydrogen atoms bonded to a silicon atom are necessary per molecule. Examples of the cross-linking agent include those compounds having two or more vinylic reactive sites per molecule, such as methylvinylpolysiloxane, α, ω-alkenyldiene, glcerintriallylether, polyoxyalkylated glycerintriallylether, trimethylolpropane triallylether, and polyoxyalkylated trimethylolpropantriallylether. Furthermore, preferably, the cross-linking organopolysiloxane contains at least a residue selected from the group consisting of polyoxyalkylene residue, polyglycerin residue, alkyl residue, alkenyl residue, aryl residue, and fluoroalkyl residue. Preferred examples are given in Japanese Laid-Open Patent Application Nos. 2-43263 and 2-214775, Japanese Patent No. 2631772, Japanese Laid-open Patent Application Nos. 9-136813(KSG30) and 2001-342255, and W003/20828 (KSG210) and W003/24413 (KSG40). These organopolysiloxanes are expected to prevent greasy look, provide matte finish, improve affinity with skin, and prevent color transferring.

The cross-linked organopolysiloxane (h) is preferably formulated in an amount of 0.1 - 30 mass %, more preferably 1 - 10 mass %, relative to the total weight of the cosmetic.

Examples of the compositions comprising the cross-linked organopolysiloxanes and the silicone oils, the hydrocarbon oils, or the ester oils include KSG-15, 16, 17, 18, 21, 210, 31, 32, 33, 34, 310, 320,330, 340, 41, 42, 43, 44, 710, 810, 820, 830, 840, which are from Shin-Etsu Chemical Co., Ltd.

Furthermore, for formulating the silicone compound of the present invention in cosmetics together with the above-described cross-linked organopolysiloxane to obtain cosmetics having high affinity with skin, it is preferable to use a homogeneous paste or gel composition comprising 100 parts by weight of the silicone compound of the present invention and 100 - 2000 parts by weight of the cross-linked organopolysiloxane.

The silicone resins (i) are gummy or solid and are soluble in decamethylcyclopentasiloxane at ambient temperatures. Preferably the gummy silicone resins are linear silicone resins represented by the general formula (CH₃)₃SiO{(CH₃)₂SiO}ᵣ{(CH₃)R³SiO}ₛSi(CH₃)₃, wherein R³ is selected from methyl group, alkyl groups having 6 - 20 carbon atoms, amino groups containing alkyl group having 3 - 15 carbon atoms, fluorine-substituted alkyl groups, and quaternary ammonium groups containing alkyl groups, r is between 1001 and 20000, s is between 1 and 5000, and r + s is between 2500 and 25000.

Solid silicone resin is preferably a network silicone compound, such as MQ resin, MDQ resin, MTQ resin, MDTQ resin, TD resin, TQ resin, and TDQ resin, which are composed of arbitrary combinations of trialkylsiloxy unit (M unit), dialkylsiloxy unit (D unit), monoalkylsiloxy unit (T unit), and tetra-functionalized siloxy unit (Q unit). Particularly preferred are network silicone compounds containing at least a residue selected from the group consisting of pyrrolidone residue, long chain alkyl residue, polyoxyalkylene residue and fluoroalkyl residue (see Japanese Laid-open Patent Application No. 2000-234062 and Japanese Patent No. 3218872).

The silicone resins (h) are preferably formulated in amount of 0.1 - 20 mass %, more preferably 1 - 10 mass %, relative to the total cosmetic amount.

The acrylic silicones are preferably semi-solid or solid at ambient temperatures. Particularly preferred are acrylic silicone resins containing at least a residue selected from the group consisting of pyyrolidone residue, long alkyl chain residue, polyoxyalkylene residue and fluoroalkyl residue. In the structure of the acrylic silicone resins, either a silicone chain or an acrylic chain may be grafted. Alternatively the acrylic silicone resins may be in a form of a silicone chain block and an acrylic chain block (see Japanese Laid-open Patent Application No. 1-319518, Japanese Patent No. 2704730, Japanese Laid-open Patent Application No. 2767633, Japanese Patent No. 2767636, Japanese Laid-open Patent Application No. 2000-344829.)

These acrylic resins or silicone resins may be formulated in cosmetics alone or in the form of dissolved product in volatile silicones, volatile hydrocarbon oils, non-volatile silicones or non-volatile hydrocarbon oils. These silicone resins may be formulated in amount of 0.1 - 20 mass %, more preferably 1 - 10 mass % , relative to the total cosmetic amount.

To obtain cosmetics with higher affinity with skin, it is desirable to use a composition comprising the acrylic silicone resin or the silicone resin in an amount of 50 - 500 parts by weight relative to 100 parts by weight of the silicone compounds of the present invention.

Examples of the UV protecting component (j) include organic UV absorbents in addition to the above-described UV scattering agents, such as inorganic pigments and metal powders. When the inorganic pigment is formulated, it is particularly preferable that the pigment is formulated in the form of dispersion in an oil agent. Examples of the dispersions using titanium oxide as a pigment and D5 as an oil agent include SPD-T1, T2, TIS, T1V, T3V, and T5, which are trade names of the products from Shi-Etsu Chemical Co., Ltd.

As for oil agents for obtaining dispersions, M3T or volatile hydrocarbon oils may be used instead of D5. In order to obtain cosmetics with higher affinity with skin, it is desirable to use a composition containing 20 - 500 parts by weight of titanium oxide relative to 100 parts by weight of the compound of the present invention.

Examples of the dispersions using zinc oxide as a pigment and D5 as an oil agent include SPD-Z1, Z2, Z3, Z1S, Z3S, Z5, which are trade names of the products from Shin-Etsu Chemical Co., Ltd. M3T or volatile hydrocarbon oils may be used instead of D5 to obtain dispersions. In order to obtain cosmetics with higher affinity with skin, it is desirable to use a composition containing 20 - 500 parts by weight of zinc oxide relative to 100 parts by weight of the compound of the present invention.

Examples of the organic ultraviolet absorbents include UV absorbents of benzoate type, such as p-aminobenzoic acid, ethyl p-aminobenzoate, glycery p-aminobenzoate, amyl p-dimethylaminobenzoate, octyl p-dimethylaminobenzoate, and ethyl 4-[N,N-di(2-hydroxypropyl)amino]benzoate; UV absorbents of salicylic acid type, such as methyl salicylate, ethyleneglycol salicylate, phenyl salicylate, octyl salicylat, benzyl salicylate, p-tert-butylphenyl salicylate, homomenthyl salicylate; those of cinnamic acid type, such as benzyl cinnamate, 2-ethoxyethyl p-methoxycinnamate, octyl p-methoxycinnamate, diparamethoxy cinnamic acid, and mono-2-ethylhexanoic acid glyceryl; UV absorbents of urocanic acid type, such as urocanic acid and ethyl urocanate, benzophenone UV absorbents, such as hydroxymethoxybenzophenon, hydroxymethoxybenzophenon sulfonic acid, sodium hydroxymethoxybenzophenon sulfonate, dihydroxymethoxybenzophenone, sodium dihydroxydimethoxybenzophenone disulfonate, 2,4-dihydroxybenzophenone, and tetrahydroxybenzophenone; UV absorbents of dibenzoylmethane type, such as 4-tert-butyl-4'-methoxydibenzoylmethane; UV absorbents of anthracitic acid type, such as homomenthyl anthranilate, benzotriazole derivatives such as 2-(2-hydroxy-5-methylphenyl)benzotriazole, and polymer derivatives thereof and silane / siloxane derivatives thereof.

The UV absorbents (j) are preferably formulated in an amount of 0.1 to 20 mass %, more preferably 1 to 10 mass %, relative to the total amount of the cosmetic. Among above-mentioned organic UV absorbents, 2-ethylhexyl p-methoxycinnamate and 4-tert-butyl-4'-methoxydibenzoylmethane are particularly preferred.

It is also possible to use an organic utltraviolet asorbents encapsulated in polymer powder. The polymer powder may be hollow or not, a mean primary particle size may be in a range of 0.1 to 50µm, and the particle distribution may be broad or sharp. Types of the polymer include acrylic resins, methacrylic resins, styrene resins, polyurethane resins, polyethylenes, polypropylenes, polyethylene terephthalates, silicone resins, nylons, and acrylamide resins. The organic utltraviolet absorbents are preferably incorporated in the polymer powder in a range from 0.1 to 30 mass% relative to the powder mass. In particular, it is desirable to use 4 - tert - butyl - 4' - methoxydibenzoylmethane which is a UVA absorbent.

In the cosmetic composition of the present invention, a variety of components that are commonly used in cosmetics can be blended in addition to the aforementioned components, as far as the effect of the present invention is not damaged, for example, film-forming agents, oil-soluble gelling agents, clay minerals modified with organic compounds, resins, moisture retention agents, antiseptics, antibacterial agents, fragrances, salts, antioxidants, pH regulators, chelating agents, refreshing agents, anti-inflammatory agents, skin beautifying components, such as skin whitener, cell activator, rough dry skin improver, blood circulation promoter, skin astringent and anti-seborrheic agent, vitamins, amino acids, nucleic acids, hormones, and clathrate compounds.

The oil-soluble gelling agent may be selected from metal soaps such as aluminum stearate, magnesium stearate, and zinc myristate; α-amino acid derivatives such as N - lauroyl-L-glutamic acid, α, γ- di - n - butylamine; dextrin fatty acid esters such as dextrin palmitate, dextrin stearate, and dextrin 2-ethylhexane palmitate; sucrose fatty acid esters such as sucrose palmitate and sucrose stearate; benzylidene derivatives of sorbitol such as monobenzylidene sorbitol and dibenzylidene sorbitol; clay minerals modified with an organic moiety such as dimethylbenzyldodecylammonium montmorillonite clay, dimethyldioctadecylammonium montmorillonite, and one or two or more gelling agents may be used, if necessary.

Examples of the moisture retention agents include glycerin, sorbitol, propylene glycol, dipropylene glycol, 1,3 - butylene glycol, pentylene glycol, glucose, xylitol, maltitol, polyethylene glycol, hyaluronic acid, chondroitin sulfuric acid, pyrrolidone carboxylate, polyoxyethylene methylglycoside, and polyoxypropylene methylglycoside.

Examples of the antiseptics indluce alkyl paraoxybenzoates, benzoic acid, sodium benzoate, sorbic acid, potassium sorbate, and phenoxyethanol may be used. For the antibacterial agents, benzoic acid, salicylic acid, carbolic acid, sorbic acid, paraoxybenzoic acid alkyl esters, parachloromethacresol, hexachlorophene, benzalkonium chloride, chlorohexydine chloride, trichlorocarbanilide, triclosan, photosensitizer, and phenoxyethanol.

Examples of the antioxidants include tocopherol, butylhydroxyanisole, dibutylhydroxytoluene and phytic acid; examples of the pH regulators include lactic acid, citric acid, glycolic acid, succinic acid, tartaric acid, dl-malic acid, potassium carbonate, sodium hydrogen carbonate and ammonium hydrogen carbonate; examples of the chelating agents include alanine, sodium ethylenediaminetetraacetate, sodium polyphosphate, sodium metaphosphate and phosphoric acid; examples of the refrigerants include L-menthol and camphor; and examples of the anti-inflammatory agents include allantoin, glycyrrhetinic acid, glycyrrhizinic acid, tranexamic acid, and azulene.

Examples of the skin-beautifying components include whitening agents, such as placenta extract, arbutin, glutathione and Yukinoshita extract; cell activators, such as royal jelly, photosensitizers, cholesterol derivatives and calf blood extract; rough and dry skin improvers; blood circulation improvers, such as nonylic acid vanillyl amide, benzyl nicotinate, beta -butoxyethyl nicotinate, capsaicin, zingerone, cantharis tincture, ichtammol, caffeine, tannic acid, alpha-borneol, tocopheryl nicotinate, inositol hexanicotinate, cyclandelate, cinnarizine, tolazoline, acetyl choline, verapamil, cepharanthin and gamma - oryzanol; skin astringents, such as zinc oxide and tannic acid; and anti-seborrheic agents, such as sulfur and thianthol; vitamins, e.g. vitamin A such as vitamin A oil, retinol, retinyl acetate and retinyl palmitate; vitamin B₂ such as riboflavin, riboflavin butyrate and flavin adenine nucleotide, vitamin B₆ such as pyridoxine hydrochloride, pyridoxine dioctanoate and pyridoxine tripalmitate, vitamin B₁₂ and its derivatives, and vitamin B₁₅ and its derivatives; vitamin C, such as L-ascorbic acid, L-ascorbate dipalmitate, sodium (L-ascorbic acid)-2-sulfate and dipotassium L-ascorbic acid diphosphate; vitamin D, such as ergocalciferol and cholecarciferol; vitamin E, such as alpha -tocopherol, beta -tocopherol, gamma -tocopherol, dl- alpha -tocopheryl acetate, dl- alpha -tocopheryl nicotinate and dl- alpha -tocopheryl succinate; vitamin H; vitamin P; nicotinic acids, such as nicotinic acid, benzyl nicotinate and nicotinic acid amide; pantothenic acids, such as calcium pantothenate, D-pantothenyl alcohol, pantothenyl ethyl ether and acetylpantothenyl ethyl ether; and biotin.

Examples of the amino acids include glycine, valine, leucine, isoleucine, serine, threonine, phenylaranine, alginine, lysine, aspartic acid, glutamic acid, cystine, cysteine, methionine, and tryptophan; examples of the nucleic acids include deoxyribonucleic acid; and examples of the hormones include estradiol and ethenyl estradiol.

Usages of the cosmetic of the present invention are not particularly limited. The following usages are most desirable: skin care products, hair care products, antiperspirant products, makeup products, UV protection products, and perfumes. Examples of these usages include basic cosmetics such as milky lotions, creams, lotions, calamine lotions, sunscreen agents, sun tanning agents, aftershave lotions, pre-shave lotions, facial pack formulas, cleansing products, facial washes, acne remedy cosmetics, and essences; makeup cosmetics such as foundation, face powder, eye shadow, eye liner, eyebrow, cheek, lipstick, and nail colors; shampoos, rinses, conditioners, hair colors, hair tonics, hair-setting agents, body powder, deodorants, hair removers, soaps, body shampoos, bath agents, hand soaps, and perfumes. The forms of the products are not particularly limited. Any forms can be selected, such as liquid form, emulsion form, cream form, solid form, paste form, gel form, powder form, multilayer form, mousse form, and spray form.

The present invention will be further explained in detail below by referring to the Examples. However, the present invention shall not be limited to these examples. "%" described below implies "% by mass" unless otherwise specified and viscosities are given as values at 25 degrees C.

### (Synthesis Example 1) Tristrimethylsiloxypropylsilane (hereinafter abbreviated as M3T-C3)

Four-necked glass flask of 2000 ml was equipped with a cooling condenser, a thermometer, and a stirrer and was purged with nitrogen, in which 649.6 g (4.0 mols) of hexamethydisiloxane and 128.0 g of methanol (4.0 mols) were placed and cooled in an ice bath until the inner temperature reached below 10 degree C. Then, 19.6 g (0.2 mols)of concentrated sulfonic acid was added dropwise over 30 minutes while the inner temperature was kept at a temperature of from 5 to 10 degrees C and the reaction mixture was stirred for another 30 minutes at the same temperature. Subsequently, 328.6 g (2.0 mols) of n-propyltrimethoxysilane was added dropwise at an inner temperature of from 5 to 10 degrees C over 45 minutes, the reaction mixture was stirred at the same temperature for 1 hour, and then 144.0 g (8.0 mols) of water was added dropwise over 2 hours at an inner temperature of from 5 to 25 degrees C.

After the completion of addition, the reaction mixture was stirred at the inner temperature of from 5 to 15 degrees C for further 3 hours. The resulting reaction mixture was allowed to stand still. An aqueous phase was separated off and the organic phase was washed with an aqueous solution of sodium bicarbonate and then with water. The resulting organic layer was distilled to obtain 608.4 g (1.8 mols) of tristrimethylsiloxy-n-propylsilane with a purity of 99.8 % as a fraction of a boiling point of from 70.0 to 70.5 degrees C / 0.5 kPa. The yield was 90.0 %.

### (Synthesis Example 2) Tristrimethylsiloxyisobutylsilane (hereinafter abbreviated as M3T-C4)

Synthesis Example 1 was repeated except that 356.0 g (2.0 mols)of isobutyltrimethoxysilane was used instead of propyltrimethoxysilane to obtain 616.0 g (1.75mols) of tristrimethylsiloxyisobutylsilane with a purity of 99.7 % as a fraction of a boiling point of from 111.0 to 111.5 degrees C / 2.9 kPa. The yield was 87.5 %.

### (Synthesis Example 3) Tristrimethylsiloxyhexylsilane (hereinafter abbreviated as M3T-C6)

Synthesis Example 1 was repeated except that 421.0 g (2.0 mols)of n-hexyltrimethoxysilane was used instead of propyltrimethoxysilane to obtain 646.0 g (1.7mols) of tristrimethylsiloxy n-hexylsilane with a purity of 99.5 % as a fraction of a boiling point of from 113.5 to 114.5 degrees C / 1.0 kPa. The yield was 85.0 %.

### (Synthesis Example 4) Tristrimethylsiloxydecylsilane (hereinafter abbreviated as M3T-C10)

Synthesis Example 1 was repeated except that 525.0 g (2.0 mols)of n-decyltrimethoxysilane was used instead of propyltrimethoxysilane to obtain 706.3 g (1.62 mols) of tristrimethylsiloxy-n-decylsilane with a purity of 98.5 % as a fraction of a boiling point of from 151.0 to 153.0 degrees C / 0.7 kPa. The yield was 81.0 %.

### [Examples 1 to 4 and Comparative Examples 1 to 3] (UV protecting cosmetic base)

A composition comprising components A to D was formulated according to the preparation method below to obtain a UV protecting makeup base. In Examples 1 to 4, the tetrasiloxanes obtained in the above Synthesis Examples 1 to 4 were used as the volatile silicone. Meanwhile in Comparative Examples 1 to 3, tristrimethylsiloxymethylsilane (M3T), octamethylcyclotetrasiloxane (D4), ordecamethylcyclopentasiloxane (D5) were used as the volatile silicone.

| (Component A) | % |
|---|---|
| (1) Silicone-treated titanium dioxide fine particle | 4 |
| (2) Volatile silicone | 10 |
| (3) KF-6017 | 1 |

| (Component B) | |
|---|---|
| (4)Silicone-treated zinc oxide fine particle | 6 |
| (5) Perfluoroalkylphosphate-treated colored skin-color mica | 0.5 |

| (Component C) | |
|---|---|
| (6) Organopolysiloxane spherical powder | 4 |
| (7) Dimethylpolysiloxane | 2 |
| (8) Fluorinated dimethiconol | 1 |
| (9) Volatile silicone | 18 |
| (10) Silicone resin (KF7312J) | 3 |
| (11) Octyl paramethoxycinnamate | 3 |
| (12) Perfluoropolyether | 0.5 |

| (Component D) | |
|---|---|
| (13) Ethyl alcohol | 10 |
| (14) Purified water | Balance |
| (15) Aloe extract (moisturizer) | 1 |
| (16) Hamamelis extract (astringent) | 1 |
| (17) Hibiscus extract (emollient component) | 0.5 |

| | |
|---|---|
| KF-6017 (from Shin-Etsu Chemical Co., Ltd.): INCI (International Nomenclatures Cosmetic Ingredient) name, PEG-10 dimethicone KF-7312J (from Shin-Etsu Chemical Co., Ltd.) : INCI name, trimethylsiloxy silicic acid / cyclopentasiloxane | |

### (Preparation Method)

- Step 1:: The ingredients for Component A were ground with a roller mill into a paste.
- Step 2:: The ingredients for Component C were simply mixed and ground well with a mixer.
- Step 3:: Components B was mixed with Component C to disperse, to which Component A was added and mixed well.
- Step 4:: The ingredients for Component D which had been dissolved homogeneously were added, stirred well, and put together with a stainless steel ball in a container to obtain a product.

The results of evaluation are as shown in the Table 1 below. As seen from the test results, the Examples of the present invention demonstrated a great affinity with skin, a longer lasting cosmetic coverage, a refreshed feel with a less greasy feel when applied and thereafter. Regarding the durability of the cosmetic coverage by the products of the Examples, the makeup run due to sebum was less. In addition, no abnormalities were observed on the skin after the application of the sample in any case.

### [Evaluation of Sensory Features and Evaluation of Durability of the Cosmetic Coverage]

Sensory features of the trial products were evaluated by a panel of ten persons. For each sensory feature, a product showing an excellent result was graded as point +5 and a product showing a poor result was graded as point 0. A product indicating a result in between these was assessed in four levels. A total score from all of the ten persons was the final valuation. Therefore, a higher score implies higher evaluation. The durability of the cosmetic coverage was assessed as follows. The durability of the cosmetic coverage was assessed as follows. After the cosmetic base obtained in one of the Examples was applied on one half of the face and that obtained in one of the Comparative Examples on the other half of the face, a commercial summer foundation was applied on the top, and the duration of the cosmetic coverage was evaluated in the same assessment method as above.

**Table 1**

| | Volatile silicone in Component A | Volatile silicone in Component C | Affinity with skin | Duration of cosmetic coverage | Non-sticky feel |
|---|---|---|---|---|---|
| Ex.1 | M3T-C3 | M3T-C3 | 45 | 44 | 44 |
| Ex. 2 | M3T-C4 | M3T-C4 | 46 | 43 | 42 |
| Ex. 3 | M3T-C6 | M3T-C6 | 43 | 41 | 42 |
| Ex.4 | M3T-C10 | M3T-C3 | 42 | 42 | 43 |
| Com. Ex. 1 | M3T | M3T | 33 | 42 | 39 |
| Com. Ex.2 | D5 | D5 | 34 | 29 | 16 |
| Com. Ex.3 | D4 | D5 | 33 | 32 | 22 |

### [Examples 6 to 8]: Whitening Cream for Daytime Use

A composition composed of Components A - B was prepared according to the following preparation method to obtain a whitening cream for daytime use.

| | Ex. 6 % | Ex. 7 % | Ex.8 % |
|---|---|---|---|
| (Component A) | | | |
| (1) KF6017 | 1 | 1 | 0 |
| (2) KF6026 | 0 | 0 | 1 |
| (3) KF56 | 5 | 5 | 5 |
| (4) M3T-C3 | 22 | 0 | 0 |
| (5) M3T-C4 | 0 | 22 | 0 |
| (6) M3T-C6 | 0 | 0 | 22 |

| (Component B) | | | |
|---|---|---|---|
| (7) Glycerin | 5 | 5 | 5 |
| (8) Dipropylene glycol | 10 | 10 | 10 |
| (9) Methyl paraoxybenzoate | 0.2 | 0.2 | 0.2 |
| (10) Sodium ascorbyl sulfate | 0.1 | 0.1 | 0.1 |
| (11) Sodium ascorbyl phosphate | 0.1 | 0.1 | 0.1 |
| (12) γ-amino butyric acid | 0.1 | 0.1 | 0.1 |
| (13) Apple seed kernel extract (antioxidant) | 0.1 | 0.1 | 0.1 |
| (14) Sodium chloride | 0.9 | 0.9 | 0.9 |
| (15) Perfume | 0.1 | 0.1 | 0.1 |
| (16) Purified water | Balance | Balance | Balance |

| | | | |
|---|---|---|---|
| KF6017 (from Shin-Etsu Chemical Co., Ltd.): INCI name, PEG-10 dimethicone KF6026 (from Shin-Etsu Chemical Co., Ltd.): INCI name, PEG / PPG -10 / 3 oleylether dimethicone KF 56 (from Shin-Etsu Chemical Co., Ltd.): INCI name, phenyl- trimethicone | | | |

### (Preparation Method)

- Step 1:: The ingredients for Component A were heated at 60°C to dissolve.
- Step 2:: The ingredients for Component B were heated at 60°C to dissolve.
- Step 3:: Component A was added to Component B with stirring to form an emulsion.
- Step 4:: Subsequently, the resulting mixture from Step 3 was cooled to 30°C with stirring and put in a container to obtain a product.

### [Comparative Example 4]

A product was obtained in the same procedures as in Example 6 except that D4 was used instead of M3T-C3.

### [Comparative Example 5]

A product was obtained in the same procedures as in Example 6 except that M3T was used instead of M3T-C3.

### [Evaluation of Sensory Features and Durability of the Cosmetic Coverage]

Sensory features of the trial products were evaluated by a panel of ten persons. A product showing an excellent result on sensory feature on questionnaire whether the feel to the touch was smooth or not was graded as point +5 and a product showing a poor result was graded as point 0, whereas one indicating a result in between these was assessed in four levels. A total score from all of the ten persons was the final valuation. Therefore, a higher score implies higher evaluation. The low-temperature stability was investigated as follows. The products were stored at 0°C and returned to room temperature. Then, it was observed visually whether any separation occurred in the products or not.

The results of the evaluation are as shown in Table 2 below. As seen from the test results, the products from the Examples of the present invention were found to have superior low-temperature stability, and an excellent affinity with skin. No abnormalities were found on the skin after the application of any one of the products.

**Table 2**

| Example | Low-temperature stability | Smooth feel to the touch | Affinity with skin |
|---|---|---|---|
| Example 6 | No problem | 45 | 42 |
| Example 7 | No problem | 43 | 44 |
| Example 8 | No problem | 43 | 46 |
| Comparative Example 4 | Separated | 39 | 33 |
| Comparative Example 5 | No problem | 43 | 36 |

### Example 9: Suntan Cream

| Components | % |
|---|---|
| 1. M3T-C3 | 15.0 |
| 2. KF96A-6 | 5.0 |
| 3. KP-562 | 0.5 |
| 4. KF6028 | 2.2 |
| 5. KF6105 | 6.0 |
| 6. Palmitic acid | 0.2 |
| 7. Dimethyloctyl paraaminobenzoic acid | 0.5 |
| 8. 4-t-butyl-4'-methoxy-dibenzoylmethane | 0.5 |
| 9. Kaoline | 0.5 |
| 10. Iron oxide red | 0.2 |
| 11. Iron oxide yellow | 0.3 |
| 12. Iron oxide black | 0.1 |
| 13. Titanium oxide coated mica | 1.0 |
| 14. Sodium L-glutamate | 3.0 |
| 15. 1,3-butylene glycol | 5.0 |
| 16. Dioctadecyldimethyl ammonium chloride | 0.1 |
| 17. Antioxidant | q.1. |
| 18. Antiseptic | q.1. |
| 19. Perfume | q.1. |
| 20. Purified water | Balance |

| | |
|---|---|
| KF96A-6 (from Shin-Etsu Chemical Co., Ltd.): INCI name, dimethicone with a viscosity of 6 mm² / sec KP-562 (from Shin-Etsu Chemical Co., Ltd.): INCI name, acrylates / behenyl acrylate / dimethicone acrylate copolymer KF6028 (from Shin-Etsu Chemical Co., Ltd.): INCI name, PEG-9 polydimethylsiloxyethyl dimethicone KF6105 (from Shin-Etsu Chemical Co., Ltd.): INCI name, Lauryl Polyglyceryl-3 Polydimethylsiloxyethyl Dimethicone | |

### (Preparation Method)

- Step 1:: Components 1 - 8 and Components 17 - 18 were dissolved while heating.
- Step 2:: After stirring Component 16 and a portion of Component 20 while heating, Components 9 - 13 were added to disperse.
- Step 3:: Components 14 - 15 and the remaining portion of Component 20 were dissolved homogeneously and combined with the resulting mixture from Step 2.
- Step 4:: While stirring, the resulting mixture from Step 3 was added portionwise to the resulting mixture from Step 1 to emulsify and cooled, to which Component 19 was added to obtain a suntan cream.

The suntan cream thus obtained could be lightly extended on the skin, had a fine texture and a non-sticky and non-greasy touch, and provided moisturized, hydrated and refreshed feel to the users. It also clung tightly to the skin and gave a long lasting coverage. It did not show quality change with temperature change or with time, such as separation or flocculation of the power, showing good stability.

### Example 10: Foundation

| Components | % |
|---|---|
| 1. M3T-C4 | 45.0 |
| 2. KF96A-6 | 5.0 |
| 3. KSG210 | 1.5 |
| 4. KF6017 | 0.5 |
| 5. Montmorillonite modified with octadecyldimethylbenzylammonium salt | 4.0 |
| 6. Titanium dioxide treated for hydrophobicity* | 10.0 |
| 7. Talc treated for hydrophobicity* | 6.0 |
| 8. Mica treated for hydrophobicity* | 6.0 |
| 9. Iron oxide red* treated for hydrophobicity | 1.6 |
| 10. Iron oxide yellow* treated for hydrophobicity | 0.7 |
| 11. Iron oxide black* treated for hydrophobicity | 0.2 |
| 12. Dipropylene glycol | 5.0 |
| 13. Methyl paraoxybenzoate | 0.3 |
| 14. 2-amino-2-methyl-1,3-propanediol | 0.2 |
| 15. Hydrochloric acid | 0.1 |
| 16. Perfume | q.1. |
| 17. Water | Balance |

| | |
|---|---|
| KF96A-6 (from Shin-Etsu Chemical Co. , Ltd. ) : INCI name, dimethicone with a viscosity of 6 mm²/sec KSG210 (from Shin-Etsu Chemical Co., Ltd.): INCI name, dimethicone, dimethicone / (PEG-10/15) crosspolymer KSG6017 (from Shin-Etsu Chemical Co., Ltd. ) : INCI name, PEG-10 dimethicone * Treatment for hydrophobicity: after adding 2% of methylhydrogenpolysiloxane to the powder, a heat treatment was applied. | |

### (Preparation Method)

- Step 1:: Components 1 - 5 were mixed while heating and Components 6 - 11 were added to obtain a homogeneous mixture.
- Step 2:: Components 12 - 15 and Component 17 were dissolved while heating (pH of the aqueous system: 9.0).
- Step 3:: While stirring, the resulting mixture from Step 2 was added portionwise to the resulting mixture from Step 1 to emulsify and cooled, to which Component 16 was added to obtain foundation.

The foundation thus obtained could be lightly extended on the skin, had a fine texture and a non-sticky and non-greasy touch, and provided moisturized, hydrated and refreshed feel to the users. The makeup coverage lasted long and it did not show quality change with temperature change or with time, showing good stability.

### Example 11: Hair Cream

| Components | % |
|---|---|
| 1. M3T-C6 | 10.0 |
| 2. KF56 | 5.0 |
| 3. Squalane | 4.0 |
| 4. Silicone resin | 1.0 |
| 5. Glyceryl dioleate | 2.0 |
| 6. KF6017 | 2.0 |
| 7. KF6026 | 4.0 |
| 8. Sodium sorbitol sulfate | 2.0 |
| 9. Sodium chondroitin sulfate | 1.0 |
| 10. Sodium hyaluronate | 0.5 |
| 11. Propylene glycol | 3.0 |
| 12. Antiseptic | 1.5 |
| 13. Vitamin E acetate | 0.1 |
| 14. Antioxidant | q.1. |
| 15. Perfume | q.1. |
| 16. Purified water | Balance |

| | |
|---|---|
| KF56 (from Shin-Etsu Chemical Co., Ltd.): Phenytrimethicone Silicone resin: 50% solution of a network silicone compound with a [Me₃SiO_{1/2}]/[SiO₂] ratio of 0.8 in M3T-C3. KF6017 (from Shin-Etsu Chemical Co., Ltd.) : INCI name, PEG-10 dimethicone. KF6026 (from Shin-Etsu Chemical Co., Ltd.): INCI name, PEG / PPG-10 / 3 oleylether dimethicone. | |

### (Preparation Method)

- Step 1:: Components 1 - 7 and Components 12 and 13 were mixed while heating.
- Step 2:: Components 8 - 11 and Component 16 were mixed to dissolve.
- Step 3:: While stirring, the resulting mixture from Step 2 was added portionwise to the resulting mixture from Step 1 to emulsify and after cooled, Component 15 was added to obtain a hair cream.

The hair cream thus obtained could be lightly extended on the skin, demonstrated a non-sticky and non-greasy touch and gave hair a moisturized and refreshed feel. It also had good water resistance and repellency, and good sweat resistance and the coverage lasted long. It showed no quality change with temperature change or with time, showing good stability.

### Example 12: Mascara

| Components | % |
|---|---|
| 1. Dissolved acrylic silicone resin | 20.0 |
| 2. Dextrin palmitate/ ethylhexanoate | 8.0 |
| 3. Polyethylene wax | 4.0 |
| 4. Beeswax | 7.0 |
| 5. Lecithin | 0.5 |
| 6. M3T-C10 | 22.0 |
| 7. C₁₁-C₁₂ Liquid isoparaffin | 20.0 |
| 8. Iron oxide | 5.0 |
| 9. AerosilRY200 | 3.5 |
| 10. Talc | 10.0 |

| | |
|---|---|
| Dissolved acrylic silicone resin: INCI name, a dissolved product of methylpolysiloxane ester of alkyl acrylates copolymer (30 %) and M3T-C3 (70%). Aerosil RY200: (from Nippon Aerosil Corp.) Hydrophobic silica | |

### (Preparation Method)

- Step 1:: Components 1 - 7 were mixed to dissolve.
- Step 2:: Components 8 - 10 were added to the resulting mixture from Step 1 and dispersed with rollers.

The mascara thus obtained could be lightly extended on the skin and demonstrated a non-sticky and non-greasy touch. It also had good water resistance and repellency and good sweat resistance and the makeup coverage lasted long. It did not show quality change with temperature change or with time, showing good stability.

### Example 13: Cream

| Components | % |
|---|---|
| 1. M3T-C3 | 20.0 |
| 2. Glyceryl trioctanoate | 10.0 |
| 3. KF6017 | 1.5 |
| 4. KF6104 | 4.0 |
| 5. Phenyldimethylstearyl ammonium chloride | 1.0 |
| 6. Dipropylene glycol | 10.0 |
| 7. Maltitol | 10.0 |
| 8. Saponite | 1.5 |
| 9. Antiseptic | q.s. |
| 10. Perfume | q.s. |
| 11. Purified water | Balance |

| | |
|---|---|
| KF6017 (from Shin-Etsu Chemical Co., Ltd.): INCI name, PEG-10 dimethicone. KF6104 (from Shin-Etsu Chemical Co., Ltd.): INCI name, polyglyceryl-3polydimethylsiloxydimethicone. | |

### (Preparation Method)

- Step 1:: Components 1 - 5 and 9 were mixed while heating.
- Step 2:: Components 6 - 8 and Component 11 were dissolved while heating.
- Step 3:: While stirring, the resulting mixture from Step 2 was added portionwise to the resulting mixture from Step 1 to emulsify and cooled, to which Component 10 was added to obtain a cream.

The cream thus obtained could be lightly extended on the skin, had a non-sticky and non-greasy touch, and provided moisturized, hydrated and refreshed feel to the users. It also had good water resistance and repellency and the makeup coverage lasted long. It showed no quality change with temperature change or with time, showing superior stability.

### Example 14: Hand cream

| Components | % |
|---|---|
| 1. M3T-C4 | 12.0 |
| 2. Liquid paraffin | 10.0 |
| 3. Dissolved silicone resin | 5.0 |
| 4. KF6017 | 1.9 |
| 5. KSG310 | 4.0 |
| 6. Distearyldimethyl ammonium chloride | 0.8 |
| 7. Vitamin E acetate | 0.1 |
| 8. Polyethylene glycol 4000 | 1.0 |
| 9. Glycerin | 10.0 |
| 10. Aluminum/magnesium silicate | 1.2 |
| 11. Antiseptic | q.1. |
| 12. Perfume | q.1. |
| 13. Purified water | Balance |

| | |
|---|---|
| Dissolved silicone resin: a dissolved product of a network silicone compound with a [Me₃SiO_{1/2}] / [SiO₂] ratio of 1.15 (50%) and M3T-C3 (50%). KF6017 (from Shin-Etsu Chemical Co., Ltd.): INCI name, PEG-10 dimethicone. KSG310 (from Shin-Etsu Chemical Co., Ltd.) : INCI name, mineral oil, PEG-15 lauryldimethicone crosspolymer. | |

### (Preparation Method)

- Step 1:: Components 1 - 7 and Component 11 were mixed while heating.
- Step 2:: Components 8 - 10 and Component 13 were mixed under heating to dissolve.
- Step 3:: While stirring, the resulting mixture from Step 2 was added portionwise to the resulting mixture from Step 1 to emulsify and cooled, to which Component 12 was added to obtain a hand cream.

The hand cream thus obtained could be extended lightly on the skin, had a non-sticky and non-greasy touch, and provided moisturized, hydrated and refreshed feel to the users. It also had good water resistance and repellency and the coverage lasted long.

### Example 15: Sunscreen Cream

| Components | % |
|---|---|
| 1. M3T-C6 | 20.0 |
| 2. Liquid paraffin | 10.0 |
| 3. KF6017 | 1.9 |
| 4. KSG-320 | 4.0 |
| 5. 4-t-butyl-4'-methoxydibenzoylmethane | 7.0 |
| 6. Distearyldimethylammonium chloride | 0.8 |
| 7. Vitamin E acetate | 0.1 |
| 8. Ethanol | 1.0 |
| 9. Aluminum / magnesium silicate | 1.2 |
| 10. Antiseptic | q.s. |
| 11. Perfume | q.s. |
| 12. Purified water | Balance |

| | |
|---|---|
| KF6017 (from Shin-Etsu Chemical Co., Ltd.): INCI name, PEG-10 dimethicone. KSG320 (from Shin-Etsu Chemical Co., Ltd.) : INCI name, isododecane, PEG-15 lauryldimethicone crosspolmer. | |

### (Preparation Method)

- Step 1:: Components 1 - 7 and 10 were mixed while heating.
- Step 2:: Components 8, 9 and 12 were heated and mixed to disperse homogeneously.
- Step 3:: While stirring, the resulting mixture from Step 2 was added portionwise to the resulting mixture from Step 1 to emulsify and cooled, to which Component 11 was added to obtain a sunscreen cream.

The sunscreen cream thus obtained could be extended lightly on the skin, had a fine texture, and provided moisturized, hydrated and refreshed feel to the users. Since it was not greasy, sands never stuck, showing very good properties for use. Since the makeup coverage lasted long, UV protecting effect also continued and it did not show quality change with temperature change or with time, showing good stability.

### Example 16: Cream

| Components | % |
|---|---|
| 1. M3T-C10 | 10.0 |
| 2. KF96A-6 | 5.0 |
| 3. Liquid paraffin | 5.0 |
| 4. KF6017 | 3.0 |
| 5. KSG340 | 5.0 |
| 6. Sodium citrate | 2.0 |
| 7. 1,3-butylene glycol | 5.0 |
| 8. Antiseptic | q.1. |
| 9. Perfume | q.1. |
| 10. Purified water | Balance |

| | |
|---|---|
| KF96A-6 (from Shin-Etsu Chemical Co. , Ltd.) : INCI name, dimethicone with a viscosity of 6 mm²/sec KF6017 (from Shin-Etsu Chemical Co., Ltd.) : INCI name, PEG-10 dimethicone. KSG340 (from Shin-Etsu Chemical Co., Ltd.) : INCI name, squalane, PEG-10 lauryldimethicone crosspolymer, PEG-15 lauryldimethicone crosspolymer. | |

### (Preparation Method)

- Step 1:: Components 1 - 4 were mixed while heating.
- Step 2:: Components 5 - 7 and Component 9 were dissolved while heating.
- Step 3:: While stirring, the resulting mixture from Step 2 was added portionwise to the resulting mixture from Step 1 to emulsify and cooled, to which Component 8 was added to obtain a cream.

The cream thus obtained could be extended lightly on the skin, had a non-sticky and non-greasy touch, and provided moisturized, hydrated and refreshed feel to the users. It also had good water resistance and repellency and the makeup coverage lasted long. It did not show quality change with temperature change or with time, showing superior stability.

### Example 17: Eye Shadow

| Components | % |
|---|---|
| 1. M3T-C3 | 15.0 |
| 2. KF96A-6 | 10.0 |
| 3. KF6009 | 2.0 |
| 4. PEG(10) laurylether | 0.5 |
| 5. Silicone-treated chromium oxide* | 6.2 |
| 6. Silicone-treated ultramarine blue* | 4.0 |
| 7. Silicone-treated titanium-coated mica* | 6.0 |
| 8. Sodium chloride | 2.0 |
| 9. Propylene glycol | 8.0 |
| 10. Antiseptic | q.1. |
| 11. Perfume | q.1. |
| 12. Purified water | Balance |

| | |
|---|---|
| KF96A-6 (from Shin-Etsu Chemical Co. , Ltd.) : INCI name, dimethicone with a viscosity of 6 mm² /sec. KF6009 (from Shin-Etsu Chemical Co., Ltd.): INCI name, PEG-9 dimethicone (silicone modified with polyether at both ends). * Silicone treatment: 3%, based on the powder, of methylhydrogenpolysiloxane was added to the powder, followed by heat treatment. | |

### (Preparation Method)

- Step 1:: Components 1 - 4 were mixed, and Component 5 - 7 were added to disperse homogeneously.
- Step 2:: Components 8 - 10 and Component 12 were dissolved homogeneously.
- Step 3:: While stirring, the resulting mixture from Step 2 was added portionwise to the resulting mixture from Step 1 to emulsify, to which Component 11 was added to obtain an eye shadow.

The eye shadow thus obtained could be extended lightly on the skin, was non-greasy and non-powdery and gave a moisturized and refreshed feel to the users. It also had good water resistance and repellency and good sweat resistance and the coverage lasted long. It did not show quality change with temperature change or with time, showing superior stability.

### Example 18 Eye Liner

| Components | % |
|---|---|
| 1. M3T-C4 | 22.0 |
| 2. KF96A-20 | 5.0 |
| 3. Jojoba oil | 2.0 |
| 4. KF6017 | 1.0 |
| 5. Silicone-treated iron oxide black * | 20.0 |
| 6. Ethanol | 5.0 |
| 7. Antiseptic | q.1. |
| 8. Purified water | Balance |

| | |
|---|---|
| KF96A-20 (from Shin-Etsu Chemical Co. , Ltd.) : INCI name, dimethicone with a viscosity of 20 mm²/sec KF6017 (from Shin-Etsu Chemical Co., Ltd.):INCI name, PEG-10 dimethicone. * Silicone-treated Iron oxide black: 2% of methylhydrogenpolysiloxane was added to iron oxide black, followed by heat treatment. | |

### (Preparation Method)

- Step 1:: Components 1 - 4 were mixed while heating, and Component 5 was added to disperse homogeneously.
- Step 2:: Components 6 - 8 were dissolved while heating.
- Step 3:: While stirring, the resulting mixture from Step 2 was added portionwise to the resulting mixture from Step 1 to emulsify to obtain an eye liner.

The eye liner thus obtained could be extended lightly on the skin, was non-greasy and non-powdery, and gave a moisturized and refreshed feel to the users. It also had good water resistance and repellency and good sweat resistance and the coverage lasted long. It did not show quality change with temperature change or with time, showing superior stability.

### Example 19 Lipcream

| Components | % |
|---|---|
| 1. M3T-C6 | 40.0 |
| 2. KSG42 | 10.0 |
| 3. Squalane | 10.0 |
| 4. Lanolin | 2.0 |
| 5. Dissolved silicone resin | 3.0 |
| 6. Micro crystalline wax | 3.0 |
| 7. KF6017 | 3.0 |
| 8. Lauroylgultamic acid dibutylamide | 5.0 |
| 9. Sodium lactate | 0.3 |
| 10. Sodium L-glutamate | 0.3 |
| 11. Sodium hyaluromate | 0.1 |
| 12. Sorbitol | 0.5 |
| 13. Glycerin | 5.0 |
| 14. Red No. 202 | q.1. |
| 15. Menthol | q.1. |
| 16. Antiseptic | q.1. |
| 17. Perfume | q.1. |
| 18. Purified water | Balance |

| | |
|---|---|
| Dissolved silicone resin: a dissolved product of a network silicone compound with a [Me₃SiO_{1/2}]/[SiO₂] ratio of 0.8 (50 %) and M3T-C3 (50%). KSG42 (from Shin-Etsu Chemical Co., Ltd.): INCI name, isododecane (vinyldimethicone / lauryldimethicone) crosspolymer KF6017 (from Shin-Etsu Chemical Co., Ltd.) : INCI name, PEG-10 dimethicone. | |

### (Preparation Method)

- Step 1:: Components 1 - 8 were mixed while heating.
- Step 2:: Components 9 - 16 and 18 were dissolved while heating.
- Step 3:: While stirring, the resulting mixture from Step 2 was added portionwise to the resulting mixture from Step 1 to emulsify, to which Component 17 was added. The mixture was put in a capsule to obtain a lipcream.

The lip cream thus obtained could be extended lightly on the skin, had a non-sticky and non-greasy touch, and provided moisturized, hydrated and refreshed feel to the users. The coverage lasted long and the treatment effect was high. It did not show quality change with temperature change or with time, showing superior stability.

### Example 20: Liquid Emulsified Foundation

| Components | % |
|---|---|
| 1. KF96A-6 | 5.0 |
| 2. M3T-C10 | 15.0 |
| 3. Squalane | 4.0 |
| 4. Neopentyl glycol dioctanoate | 3.0 |
| 5. Myristic acid isostearic acid diglyceride | 2.0 |
| 6. α-monoisostearyl glycerylether | 1.0 |
| 7. KSG710 | 1.0 |
| 8. Aluminum distearate | 0.2 |
| 9. Titanium dioxide treated for hydrophobicity* | 5.0 |
| 10. Cerisite treated for hydrophobicity* | 2.0 |
| 11. Talc treated for hydrophobicity* | 3.0 |
| 12. Iron oxide red treated for hydrophobicity* | 0.4 |
| 13. Iron oxide yellow treated for hydrophobicity* | 0.7 |
| 14. Iron oxide black treated for hydrophobicity* | 0.1 |
| 15. Magnesium sulfate | 0.7 |
| 16. Glycerin | 3.0 |
| 17. Antiseptic | q.1. |
| 18. Perfume | q.1. |
| 19. Purified water | Balance |

| | |
|---|---|
| KF96A-6 (from Shin-Etsu Chemical Co. , Ltd.) : INCI name, dimethicone with a viscosity of 6 mm² /sec. KSG710 (from Shin-Etsu Chemical Co., Ltd.) : INCI name, a composition comprising cross-linked glycerin-modified silicone and dimethicone. * Hydrophobic powder: powder was treated with 2%, based on the powder, of stearic acid. | |

### (Preparation Method)

- Step 1:: Components 1 - 8 were mixed while heating and Components 9 - 14 were added to obtain a homogeneous mixture.
- Step 2:: Components 15 - 17 and Component 19 were dissolved while heating.
- Step 3:: While stirring, the resulting mixture from Step 2 was added portionwise to the resulting mixture from Step 1 to emulsify and cooled, to which Component 18 was added to obtain a liquid emulsified foundation.

The liquid emulsified foundation thus obtained was less viscous, could be extended lightly on the skin, had a fine texture, a non-sticky and non-greasy touch, provided moisturized, hydrated and refreshed feel to the users, and the makeup coverage lasted long. It did not show quality change with temperature change or with time, showing superior stability.

### Example 21: Antiperspirant

| Components | % |
|---|---|
| 1. M3T-C3 | 30.0 |
| 2. KF6026 | 1.0 |
| 3. Polyoxyethylenesorbitan monooleate (20 E.O.) | 0.5 |
| 4. Aluminum zirconium tetrachlorohydrate glycine salt | 20.0 |
| 5. Purified water | Balance |

| | |
|---|---|
| KF6026 (from Shin-Etsu Chemical Co., Ltd.): INCI name PEG / PPG-10 / 3oleylether dimethicone. | |

### (Preparation Method)

- Step 1:: Components 1 and 2 were mixed.
- Step 2:: Component 4 was dissolved in Component 5, to which Component 3 was added.
- Step 3:: While stirring, the resulting mixture from Step 2 was added portionwise to the resulting mixture from Step 1 to emulsify to obtain an antiperspirant.

The antiperspirant thus obtained could be extended lightly on the skin, had a non-sticky and non-greasy touch, did not leave too much white powdery residue, and gave a refreshed feel to the users. It did not show quality change with temperature change or with time, showing superior stability.

### Example 22: Transparent Gel Cosmetic

| Components | % |
|---|---|
| 1. M3T-C4 | 10.0 |
| 2. KF6100 | 10.0 |
| 3. 1,3-butylene glycol | 10.0 |
| 4. Polyethylene glycol 400 | 9.0 |
| 5. 2-hydroxyoctanoic acid | 1.0 |
| 6. Sorbitol (70% aqueous solution) | 10.0 |
| 7. Citric acid | q.1. |
| 8. Sodium citrate | q.1. |
| 9. Antiseptic | q.1. |
| 10. Perfume | q.1. |
| 11. Purified water | Balance |

| | |
|---|---|
| KF6100 (from Shin-Etsu Chemical Co., Ltd.): INCI name, polyglyceryl-3 disiloxane dimethicone. | |

### (Preparation Method)

- Step 1:: Components 3 - 11 were dissolved homogeneously.
- Step 2:: Components 1 and 2 were mixed to obtain a homogeneous mixture.
- Step 3:: While stirring, the resulting mixture from Step 1 was added portionwise to the resulting mixture from Step 2 to emulsify to obtain a transparent gel cosmetic.

The transparent gel cosmetic thus obtained could be lightly extended on the skin, had a non-sticky and non-greasy touch, and gave the users a moisturized, hydrated and refreshed feel. It did not show quality change with temperature change or with time, showing superior stability.

### Example 23: Sunscreen Lotion

| Components | % |
|---|---|
| 1. M3T-C6 | 14.0 |
| 2. Silicone composition | 10.0 |
| 3. Squalane | 1.5 |
| 4. Octyl paramethoxycinnmate | 3.0 |
| 5. Titanium TTO - S2 | 2.0 |
| 6. 1,3-butylene glycol | 10.0 |
| 7. Sodium chloride | 2.0 |
| 8. L-proline | 0.1 |
| 9. 2-hydroxyoctanoic acid | 1.0 |
| 10. 2-hydroxypropanoic acid | 5.0 |
| 11. Sodium hydroxide | q.1. |
| 12. Antiseptic | q.1. |
| 13. Perfume | q.1. |
| 14. Purified water | Balance |

| | |
|---|---|
| Silicone composition: a composition comprising cross-linked dimethylpolysiloxane and M3T-C3. Titanium TTO-S2 (from Sakai Chemical Industry Co., Ltd.): titanium dioxide treated for hydrophobicity. | |

### (Preparation Method)

- Step 1:: Components 6 - 14 were dissolved homogeneously.
- Step 2:: Components 1 - 4 were mixed, to which Component 5 was added to obtain a homogeneous mixture.
- Step 3:: While stirring, the resulting mixture from Step 2 was added portionwise to the resulting mixture from Step 1 to emulsify to obtain a sunscreen lotion.

The sunscreen lotion thus obtained could be extended lightly on the skin, had a non-sticky and non-greasy touch and a high affinity with skin, and gave the users moisturized, hydrated and refreshed feel. The effect as sunscreen agent was also high and no quality change was found with temperature change or with time.

### Example 24: Cream

| Components | % |
|---|---|
| 1. M3T-C10 | 20.0 |
| 2. Liquid paraffin | 5.0 |
| 3. KSG310 | 1.0 |
| 4. Magnesium L-ascorbate phosphate | 3.0 |
| 5. Dipropylene glycol | 5.0 |
| 6. Glycerin | 5.0 |
| 7. Antiseptic | q.1. |
| 8. Perfume | q.1. |
| 9. Purified water | Balance |

| | |
|---|---|
| KSG310 (from Shin-Etsu Chemical Co., Ltd.): INCI name, PEG-5 lauryldimethicone crosspolymer / mineral oil. | |

### (Preparation Method)

- Step 1:: Components 1 - 3 were mixed homogeneously.
- Step 2:: Components 5 - 7 were heated and made homogeneous.
- Step 3:: Components 4 and 9 were dissolved homogeneously.
- Step 4:: While stirring, the resulting mixture from Step 2 was added portionwise to the resulting mixture from Step 1 and then the resulting mixture from Step 3 was added to emulsify, to which Component 8 was added to obtain a cream.

The cream thus obtained could be lightly extended on the skin, had a non-sticky and non-greasy touch, and provided moisturized, hydrated and refreshed feel to the users. It also had a high affinity with skin and the whitening effect was high. It did not show quality change with temperature change or with time, showing superior stability.

### Example 25 Eye Liner

| Components | % |
|---|---|
| 1. M3T-C10 | 53.5 |
| 2. KF6105 | 3.0 |
| 3. KF7312J | 15.0 |
| 4. Dimethylstearyammonium hectolite | 3.0 |
| 5. Silicone-treated iron oxide black | 10.0 |
| 6. 1,3 - butylene glycol | 5.0 |
| 7. Sodium sulfate | 0.5 |
| 8. Antiseptic | q.1. |
| 9. Purified water | Balance |

| | |
|---|---|
| KF7312J (from Shin-Etsu Chemical Co. , Ltd.) : INCI name, trimethylsiloxy silicate, cyclopentasiloxane (a dissolved product of a network silicone compound (50%) and D5 (50 %)). Silicone-treated Iron oxide black: 2% of methylhydrogenpolysiloxane was added to iron oxide black, followed by a heat treatment. | |

### (Preparation Method)

- Step 1:: Components 1 - 4 were mixed while heating, and Component 5 was added to disperse homogeneously.
- Step 2:: Components 6 - 9 were mixed.
- Step 3:: While stirring, the resulting mixture from Step 2 was added portionwise to the resulting mixture from Step 1 to obtain an eye liner.

The eye liner thus obtained could be lightly extended on the skin, was easy to draw, had a refreshing feel and a lightweight and non-sticky touch. It did not show quality change with temperature change or with time, showing superior properties for use and stability. It was also confirmed that it had good water resistance as well as sweat resistance and the makeup coverage lasted long.

### Example 26: Milky Lotion

| Components | % |
|---|---|
| 1. M3T-C4 | 15.0 |
| 2. KF96A-6 | 6.0 |
| 3. Squalan | 5.0 |
| 4. Neopentylglycol dioctanoate | 3.0 |
| 5. α-monooleylglyceryl ether | 1.0 |
| 6. KSG830 | 1.5 |
| 7. KF6017 | 1.0 |
| 8. Aluminum distearate | 0.2 |
| 9. Dextrin fatty acid ester | 1.0 |
| 10. Magnesium sulfate | 0.7 |
| 11. Glycerin | 5.0 |
| 12. Antiseptic | q.1. |
| 13. Perfume | q.1. |
| 14. Purified water | Balance |

| | |
|---|---|
| KF96A-6 (from Shin-Etsu Chemical Co., Ltd.): INCI name, dimethicone with a viscosity of 6 mm²/sec. KF830 (from Shin-Etsu Chemical Co., Ltd.): INCI name, polyglyceryl-3lauryldimethicone crosspolymer / trioctanoin KF6017 (from Shin-Etsu Chemical Co., Ltd.): INCI name, PEG-10 dimethicone. | |

### (Preparation Method)

- Step 1:: Components 1 - 9 were mixed while heating.
- Step 2:: Components 10 - 12 and Component 14 were dissolved while heating.
- Step 3:: While stirring, the resulting mixture from Step 2 was added portionwise to the resulting mixture from Step 1 to emulsify and cooled, to which Component 13 was added to obtain a milky lotion.

The milky lotion thus obtained could be lightly extended on the skin, had a low viscosity, a fine texture, a non-sticky and non-greasy touch, and gave the users moisturized, hydrated, and refreshed feel. The cosmetic coverage lasted long and no quality change was found with temperature change or with time.

### Example 27: Sunscreen Cream

| Components | % |
|---|---|
| 1. M3T-C6 | 18.0 |
| 2. KF56 | 2.0 |
| 3. Liquid paraffin | 1.5 |
| 4. KF6009 | 4.0 |
| 5. Octyl paramethoxycinnamate | 5.0 |
| 6. 1,3-butylene glycol | 4.0 |
| 7. Sodium chloride | 1.0 |
| 8. Antiseptic | q.1. |
| 9. Perfume | q.1. |
| 10. Purified water | balance |

| | |
|---|---|
| KF56 (from Shin-Etsu Chemical Co., Ltd.): INCI name, phenyltrimethicone KF6009 (from Shin-Etsu Chemical Co., Ltd.): INCI name, PEG-9 Dimethicone (silicone modified with polyether at both ends). | |

### (Preparation Method)

- Step 1:: Components 1 - 5 were mixed while heating.
- Step 2:: Components 6 - 8 and 10 were heated to dissolve.
- Step 3:: While stirring, the resulting mixture from Step 2 was added portionwise to the resulting mixture from Step 1 and cooled, to which Component 9 was added to obtain a sunscreen cream.

The sunscreen cream thus obtained could be lightly extended on the skin, had a fine texture, and provided moisturized, hydrated and refreshed feel to the users. It had good water resistance and sweat resistance and the makeup coverage lasted long. UV protecting effect also continued and it did not show quality change with temperature change or with time, showing superior stability.

### Example 28: Cream

| Components | % |
|---|---|
| 1. M3T-C10 | 20.0 |
| 2. KF56 | 5.0 |
| 3. KSG330 | 1.0 |
| 4. Dextrin fatty acid ester | 1.0 |
| 5. Glycerin | 5.0 |
| 6. Sodium chloride | 1.0 |
| 7. Antiseptic | q.1. |
| 8. Perfume | q.1. |
| 9. Purified water | Balance |

| | |
|---|---|
| KF56 (from Shin-Etsu Chemical Co., Ltd.): INCI name, phenyltrimethicone. KSG330 (from Shin-Etsu Chemical Co., Ltd.): INCI name, trioctanoin, PEG-15 lauryldimethicone crosspolmer. | |

### (Preparation Method)

- Step 1:: Components 1 - 4 were mixed while heating.
- Step 2:: Components 5 - 7 and 9 were heated to dissolve.
- Step 3:: While stirring, the resulting mixture from Step 2 was added portionwise to the resulting mixture from Step 1 to emulsify and cooled, to which Component 8 was added to obtain a cream.

The cream thus obtained could be lightly extended on the skin, had a fine texture and a non-sticky and non-greasy touch, and gave moisturized and hydrated feel. It also had good water resistance and sweat resistance and the makeup coverage lasted long. It did not show quality change with temperature change or with time, showing good stability.

### Example 29: Foundation

| Components | % |
|---|---|
| 1. M3T-C3 | 18.0 |
| 2. KF56 | 5.0 |
| 3. Sorbitan monoisostearate | 0.5 |
| 4. Digylceryl monoisostearate | 0.5 |
| 5. KP561 | 1.0 |
| 6. Octyl paramethoxycinnamate | 3.0 |
| 7. Titanium oxide | 10.0 |
| 8. Iron oxide red | 0.13 |
| 9. Iron oxide yellow | 0.3 |
| 10. Iron oxide black | 0.07 |
| 11. Talc | 2.5 |
| 12. Sorbitol | 2.0 |
| 13. Maganesium sulfate | 0.1 |
| 14. Ethanol | 10.0 |
| 15. Antiseptic | q.1. |
| 16. Perfume | q.1. |
| 17. Water | Balance |

| | |
|---|---|
| KF56 (from Shin-Etsu Chemical Co., Ltd.) : INCI name, phenyltrimethicone. KP561 (from Shin-Etsu Chemical Co., Ltd.) : INCI name, (alkyl acrylates / stearyl acrylate / dimethicone acrylate) copolymer. | |

### (Preparation Method)

- Step 1:: Components 7 - 11 were mixed homogeneously.
- Step 2:: Components 1 - 6 and Component 15 were mixed while heating, and the resulting mixture from Step 1 was added to disperse homogeneously
- Step 3:: Components 12 - 13 and 17 were heated, and the resulting mixture was added to the resulting mixture from Step 2 to emulsify and cooled, to which Components 14 and 16 were added to obtain a foundation.

The foundation thus obtained could be lightly extended on the skin, had a non-sticky touch and a long lasting UV protecting effect as well as lightweight refreshing feel. The resulting emulsion state was also very good. It was not susceptible to temperature change, i.e. it did not cause separation or flocculation with time, showing superior stability.

### Example 30: Liquid Foundation

| Components | % |
|---|---|
| 1. M3T-C4 | 15.0 |
| 2. KF96A-6 | 5.0 |
| 3. Liquid paraffin | 3.0 |
| 4. KF6015 | 3.0 |
| 5. Palmitic acid | 0.5 |
| 6. AerosilRY200 | 5.0 |
| 7. Titanium dioxide | 6.0 |
| 8. Iron oxide red | 0.25 |
| 9. Iron oxide yellow | 0.6 |
| 10. Iron oxide black | 0.12 |
| 11. Sericite | 8.03 |
| 12. Dipropyleneglycol | 10.0 |
| 13. Magnesium sulfate | 2.0 |
| 14. Antiseptic | q.1. |
| 15. Antioxidant | q.1. |
| 16. Perfume | q.1. |
| 17. Purified water | Balance |

| | |
|---|---|
| KF96A-6 (from Shin-Etsu Chemical Co., Ltd.): INCI name, dimethicone with a viscosity of 6 mm² /sec. KF6015 (from Shin-Etsu Chemical Co., Ltd.): INCI name, PEG-3dimethicone. Aerosil RY200 (from Nippon Aerosil Corp.): hydrophobic silica. | |

### (Preparation Method)

- Step 1:: Components 8 - 11 were mixed homogeneously.
- Step 2:: Components 1 - 7 and Component 15 were heated to 70 degrees C, to which the resulting mixture from Step 1 was added to disperse homogeneously.
- Step 3:: Components 12 - 17 were heated to 70 degrees C, which was added to the resulting mixture from Step 2 to emulsify and cooled, to which Component 16 was added to obtain a liquid foundation.

The liquid foundation thus obtained could be lightly on the skin, had a non-sticky and light touch, and gave a refreshed feel. It also had a very good emulsion state and the makeup coverage lasted long. It was not susceptible to temperature change, showing superior stability with time.

### Example 31: Sunscreen Milky Lotion

| Components | % |
|---|---|
| 1. M3T-C4 | 25.0 |
| 2. Diglyceryl monoisostearate | 1.5 |
| 3. Decaglyceryl pentaisostearate | 1.5 |
| 4. KF6017 | 0.5 |
| 5. Olive oil | 1.0 |
| 6. SPD-T5 | 7.0 |
| 7. Glycerin | 5.0 |
| 8. Sodium chloride | 1.5 |
| 9. Antiseptic | q.1. |
| 10. Perfume | q.1. |
| 11. Purified water | Balance |

| | |
|---|---|
| KF6017 (from Shin-Etsu Chemical Co., Ltd.) : INCI name, PEG-10 dimethicone. SPD-T5 (from Shin-Etsu Chemical Co., Ltd.): INCI name, stearic acid, cyclopentasiloxane, polyglyceryl-3 / polydimethylsiloxyethyldimethicone, Al hydroxide, titanium oxide. | |

### (Preparation Method)

- Step 1:: Components 1 - 5 were mixed while heating and Component 6 was dispersed homogeneously.
- Step 2:: Components 7 - 9 and 11 were mixed while heating.
- Step 3:: While stirring, the resulting mixture from Step 2 was added portionwise to the resulting mixture from Step 1 to emulsify and cooled, to which Component 10 was added to obtain a sunscreen milky lotion.

The sunscreen milky lotion thus obtained could be lightly extended on the skin, had a low viscosity, a fine texture, and a non-sticky touch and provided moisturized, hydrated feel to the users. Since the makeup coverage lasted long, UV protecting effect also continued. The stability of the powder dispersion and stability of the emulsion were also very good.

### Example 32: Sunscreen Milky Lotion

| Components | % |
|---|---|
| 1. M3T-C6 | 20.0 |
| 2. KF56 | 3.0 |
| 3. Sorbitan monoisostearate | 1.0 |
| 4. KF6012 | 0.5 |
| 5. SPD-T5 | 5.0 |
| 6. Octyl paramethoxycinnamate | 4.0 |
| 7. SPD-D5 | 8.0 |
| 8. Sorbitol | 2.0 |
| 9. Sodium chloride | 2.5 |
| 10. Antiseptic | q.1. |
| 11. Perfume | q.1. |
| 12. Purified water | Balance |

| | |
|---|---|
| KF56 (from Shin-Etsu Chemical Co., Ltd.): INCI name, phenyltrimethicone SPD-T5 (from Shin-Etsu Chemical Co., Ltd.): INCI name, stearic acid, cyclopentasiloxane, polygylceryl-3 / polydimethylsiloxyethyldimethicone, Al hydroxide, Titanium oxide KF6012 (from Shin-Etsu Chemical Co., Ltd.): INCI name, PEG / PPG - 20 / 22 butylether dimethicone SPD-Z5 (from Shin-Etsu Chemical Co., Ltd.) : INCI name, Zinc Oxide, Cyclopentasiloxane, Polyglyceryl-3 Polydimethylsiloxyethyl Dimethicone, Hexyl Triethoxy silylethyl Polydimethylsiloxyethyl Dimethicone. | |

### (Preparation Method)

- Step 1:: Components 1 - 7 were mixed while heating.
- Step 2:: Components 8 - 10 and 12 were mixed while heating.
- Step 3:: While stirring, the resulting mixture from Step 2 was added portionwise to the resulting mixture from Step 1 to emulsify and cooled, to which Component 11 was added to obtain a sunscreen milky lotion.

The sunscreen milky lotion thus obtained could be lightly extended on the skin, had a fine texture and a non-sticky touch, and provided moisturized, hydrated feel to users. Since the makeup coverage lasted long, UV protecting effect also continued. It did not show quality change with temperature change or with time, showing good stability.

### Example 33: Beautifying Liquid

| Components | % |
|---|---|
| 1. M3T-C10 | 12.0 |
| 2. Glyceryl triisooctanoate | 10.0 |
| 3. KF6017 | 0.2 |
| 4. KSG21 | 2.0 |
| 5. Glycerin | 10.0 |
| 6. Magnesium ascorbate phosphate | 3.0 |
| 7. Sodium chloride | 2.0 |
| 8. Antiseptic | q.1. |
| 9. Perfume | q.1. |
| 10. Purified water | Balance |

| | |
|---|---|
| KF6017 (from Shin-Etsu Chemical Co., Ltd.) : INCI name, PEG-10 dimethicone KSG21 (from Shin-Etsu Chemical Co., Ltd.): INCI name PEG-10 dimethicone crosspolymer, dimethicone | |

### (Preparation Method)

- Step 1:: Components 1 - 4 were mixed while.
- Step 2:: Components 5 - 8 and Components 10 were heated to dissolve homogeneously.
- Step 3:: While stirring the resulting mixture from Step 2 was added portionwise to the resulting mixture from Step 1 to emulsify to obtain a beautifying liquid.

The beautifying liquid thus obtained could be lightly extended on the skin, had a fine texture and a non-sticky touch, and provided moisturized and non-sticky feel to users. It did not show quality change with temperature change or with time, showing good stability.

### Example 34: Cream

| Components | % |
|---|---|
| 1. M3T-C3 | 18.0 |
| 2. KF96A-100 | 2.0 |
| 3. Polypropyleneglycol (3) myristyl ether | 0.5 |
| 4. KF6017 | 1.4 |
| 5. KF6105 | 2.5 |
| 6. Titanium dioxide treated for hydrophobicity* | 1.0 |
| 7. Glycerin | 3.0 |
| 8. 70 % Sorbitol | 5.0 |
| 9. Citric acid | 25.0 |
| 10. Sodium chloride | 0.6 |
| 11. Antiseptic | q.1. |
| 12. Perfume | q.1. |
| 13. 32 % aqueous ammonium | 4.5 |
| 14. Purified water | Balance |

| | |
|---|---|
| KF96A-100 (from Shin-Etsu Chemical Co., Ltd.): INCI name, dimethicone with a viscosity of 100 mm²/sec KF6017 (from Shin-Etsu Chemical Co., Ltd.): INCI name, PEG-10 dimethicone KF6015 (from Shin-Etsu Chemical Co., Ltd.): INCI name,Lauryl Polyglyceryl-3 Polydimethylsiloxyethyl Dimethicone * Titanium dioxide treated for hydrophobicity: fine particle of titanium oxide treated with aluminum stearate. | |

### (Preparation Method)

- Step 1:: Components 1 - 5 and 12 were mixed while heating and Component 6 was added to obtain a homogeneous mixture.
- Step 2:: Components 7 - 11 and Components 13 - 14 were dissolved homogeneously.
- Step 3:: The resulting mixture from Step 2 was added portionwise to the resulting mixture from Step 1 to emulsify to obtain a cream.

The cream thus obtained could be lightly extended on the skin when applied and had a non-sticky touch in spite of a large content of citric acid. It also left skin moisturized feel after application. It did not show quality change with temperature change or with time, showing good stability.

### Example 33: Aftershave Cream

| Components | % |
|---|---|
| 1. M3T-C4 | 35.0 |
| 2. KF6017 | 2.9 |
| 3. KF6026 | 5.0 |
| 4. Polyethylene glycol (molecular weight: 400) | 5.0 |
| 5. Sodium L-glutamate | 2.0 |
| 6. Arantoin | 0.1 |
| 7. Aloe extract | q.1. |
| 8. Antiseptic | q.1. |
| 9. Antioxidant | q.1. |
| 10. Perfume | q.1. |
| 11. Purified water | Balance |

| | |
|---|---|
| KF6017 (from Shin-Etsu Chemical Co., Ltd.): INCI name, PEG-10 dimethicone KF6026 (from Shin-Etsu Chemical Co., Ltd.): INCI name, PEG / PPG-10 / 3oleylether dimethicone. | |

### (Preparation Method)

- Step 1:: Components 1 - 4 and Components 10 and 11 were mixed while heating.
- Step 2:: Components 5 - 9 were mixed while heating.
- Step 3:: The resulting mixture from Step 2 was added portionwise to the resulting mixture from Step 1 to emulsify to obtain an aftershave cream.

The aftershave cream thus obtained was viscous, did not cause sagging, could be lightly extended on the skin in use and was non-sticky. After application it maintained a light touch and moisturizing feel and demonstrated superior stability.

### Example 36: Deodorant

| Components | % |
|---|---|
| 1. M3T-C6 | 12.0 |
| 2. KF96A-6 | 4.0 |
| 3. KF6026 | 1.0 |
| 4. Propylene glycol | 31.0 |
| 5. Triclosan | 0.1 |
| 6. Glycerin | 15.0 |
| 7. Antiseptic | q.1. |
| 8. Perfume | q.1. |
| 9. Purified water | Balance |

| | |
|---|---|
| KF96A-6 (from Shin-Etsu Chemical Co., Ltd.) : INCI name, dimethicone with a viscosity of 6 mm² / sec. KF6026 (from Shin-Etsu Chemical Co., Ltd.): INCI name, PEG / PPG-10 / 3-oleyl ether dimethicone. | |

### (Preparation Method)

- Step 1:: Components 1 - 3 were mixed.
- Step 2:: Components 5 was dissolved in Component 4, and then Components 6 - 9 were mixed.
- Step 3:: Under a vigorous stirring, the resulting mixture from Step 2 was added to the resulting mixture from Step 1 to emulsify.
- Step 4:: 65 parts by weight of the resulting mixture from Step 3 and 35 parts by weight of a propellant (a mixture of n-butane, isobutane, and propane) were put in an aerosol can to obtain a deodorant.

The deodorant thus obtained demonstrated no sagging when used at a high concentration and was non-sticky and light. It provided a long lasting effect, showing good properties for use.

### Example 37: Liquid Foundation

| Components | % |
|---|---|
| 1. M3T-C10 | 16.0 |
| 2. KF96A-6 | 8.0 |
| 3. Octyl paramethoxycinnamate | 3.0 |
| 4. 12-hydroxysteatic acid | 1.0 |
| 5. FL-5 | 15.0 |
| 6. FPD-6131 | 5.0 |
| 7. KSP101 | 3.0 |
| 8. Fine particle titanium oxide treated with a fluorine compound* | 8.0 |
| 9. Mica titanium treated with a fluorine compound* | 1.0 |
| 10. Titanium oxide treated with a fluorine compound* | 5.0 |
| 11. Iron oxide red treated with a fluorine compound* | 0.9 |
| 12. Iron oxide yellow treated with a fluorine compound* | 2.0 |
| 13. Iron oxide black treated with a fluorine compound* | 1.0 |
| 14. Ethanol | 15.0 |
| 15. Glycerin | 3.0 |
| 16. Magnesium sulfate | 1.0 |
| 17. Antiseptic | q.1. |
| 18. Perfume | q.1. |
| 19. Purified water | Balance |

| | |
|---|---|
| KF96A-6 (from Shin-Etsu Chemical Co. , Ltd.) : INCI name, dimethicone with a viscosity of 6 mm²/sec. FL-5 (from Shin-Etsu Chemical Co., Ltd.): INCI name, Trifluoropropyldimethicone with a viscosity of 10 mm²/sec FPD-6131 (from Shin-Etsu Chemical Co., Ltd.) : INCI name, PEG-8 trifluoropropyldimethicone polymer. KSP101 (from Shin-Etsu Chemical Co., Ltd.): INCI name, (dimethicone / vinyldimethicone / methicone) cross-polymer. * Treatment with a fluorine compound: coated with 5% of perfluoroalkylethylphosphate diethanolamine salt | |

### (Preparation Method)

- Step 1:: Components 7 - 13 were mixed homogeneously.
- Step 2:: Components 1 - 6 were mixed while heating to 70 degrees C, to which the resulting mixture from Step 1 was added to obtain a homogeneous dispersion.
- Step 3.: Components 14 - 17 and Component 19 were heated to 40 degrees C and added portionwise to the resulting mixture from Step 2 to emulsify and cooled, to which Component 18 was added to obtain a liquid foundation.

The foundation thus obtained could be lightly extended on the skin, was non-sticky, and gave a refreshing feel to the users. It did not show quality change with temperature change or with time, showing superior stability.

### Example 38: Milky Lotion

| Components | % |
|---|---|
| 1. M3T-C3 | 15.0 |
| 2. KF56 | 5.0 |
| 3. Squalene | 5.0 |
| 4. Pentaerythritol tetra-2-ethylhexanoate | 5.0 |
| 5. KF6017 | 3.0 |
| 6. KSP101 | 2.0 |
| 7. Aerosil R972 | 0.5 |
| 8. Magnesium ascorbate | 1.0 |
| 9. Sodium chloride | 1.0 |
| 10. Polyethylene glycol 11000 | 1.0 |
| 11. Propylene glycol | 8.0 |
| 12. Antiseptic | q.1. |
| 13. Perfume | q.1. |
| 14. Purified water | balance |

| | |
|---|---|
| KF56 (from Shin-Etsu Chemical Co., Ltd.): INCI name, phenyltrimethicone KF6017 (from Shin-Etsu Chemical Co., Ltd.): INCI name, PEG-10 dimethicone KSP101 (from Shin-Etsu Chemical Co., Ltd.) : INCI name, (dimethicone / vinyldimethicone / methicone) cross-polymer Aerosil R972 (from Nippon Aerosil Corp.): Hydrophobic silica | |

### (Preparation Method)

- Step 1:: Components 1 - 5 were mixed homogeneously and Components 6 - 7 were added to disperse homogeneously.
- Step 2:: Components 8 - 10 were added to Component 14 to dissolve, to which a homogeneous mixture of Components 11 and 12, was added.
- Step 3:: The resulting mixture from Step 2 was added portionwise to the resulting mixture from Step 1 to emulsify and cooled, to which Component 13 was added to obtain a milky lotion.

The milky lotion thus obtained could be lightly extended on the skin, was light and non-sticky, and did not show quality change with temperature change or with time, showing superior stability and properties for use.

### Example 39: Moisturizing Cream

| Components | % |
|---|---|
| 1. M3T-C4 | 10.0 |
| 2. KSG16 | 3.0 |
| 3. KSG310 | 5.0 |
| 4. Pentaerythritol tetra-2-ethylhexanoate | 3.0 |
| 5. Cetyl 2-ethylhexanoate | 5.0 |
| 6. KF6017 | 1.0 |
| 7. KSP300 | 2.5 |
| 8. Aerosil R972 | 2.0 |
| 9. Zinc stearate | 2.0 |
| 10. Vitamin E acetate | 3.0 |
| 11. Polyethylene glycol 400 | 1.0 |
| 12. Sodium lactate | 1.0 |
| 13. 1,3-butylene glycol | 5.0 |
| 14. Antiseptic | q.1. |
| 15. Perfume | q.1. |
| 16. Purified water | Balance |

| | |
|---|---|
| KSG310 (from Shin-Etsu Chemical Co., Ltd. ) : INCI name, mineral oil, PEG-15 Lauryldimethicone crosspolymer. KSG16 (from Shin-Etsu Chemical Co., Ltd.): INCI name, dimethicone, (dimethicone / vinyldimethicone) crosspolymer. KF6017 (from Shin-Etsu Chemical Co., Ltd.) : INCI name, PEG-10, dimethicone. KSP300 (from Shin-Etsu Chemical Co., Ltd.) : INCI name, (diphenyldimethicone / vinyldiphenyldimethicone / silsesquioxane) crosspolymer. AerosilR972 (from Nippon Aerosil Corp.): hydrophobic silica. | |

### (Preparation Method)

- Step 1:: Components 1 - 6 and Components 9 - 10 were mixed homogeneously, to which Components 7 - 8 were added to disperse.
- Step 2:: Components 11 - 14 and Component 16 were combined and dissolve.
- Step 3:: The resulting mixture from Step 2 was added portionwise to the resulting mixture from Step 1 to emulsify and cooled, to which Component 15 was added to obtain a moisturizing cream.

The moisturizing cream thus obtained could be extended lightly on the skin, was hydrating, light and non-sticky, and presented no quality changes with temperature change or with time, showing superior stability and properties for use.

### Example 40: Hand cream

| Components | % |
|---|---|
| 1. M3T-C6 | 30.0 |
| 2. Liquid paraffin | 10.0 |
| 3. Gummy amino-modified silicone | 15.0 |
| 4. KF6017 | 4.0 |
| 5. Distearyldimethyl ammonium chloride | 0.8 |
| 6. Vitamin E acetate | 0.1 |
| 7. Polyethylene glycol 4000 | 1.0 |
| 8. Glycerin | 10.0 |
| 9. Aluminum magnesium silicate | 1.2 |
| 10. Antiseptic | q.1. |
| 11. Perfume | q.1. |
| 12. Purified water | Balance |

| | |
|---|---|
| Gummy amino-modified silicone: gummy dimethicone modified with aminopropyl (amine equivalent weight;70000g/mol, viscosity; 30,000,000 mm² /sec) KF6017 (from Shin-Etsu Chemical Co., Ltd.): INCI name, PEG-10 dimethicone. | |

### (Preparation Method)

- Step 1:: Components 1 and 3 were mixed to dissolve while heating, and Components 2, 4 - 6 and 10 were added while heating.
- Step 2:: Components 7 - 9 and Component 12 were mixed under heating.
- Step 3:: The resulting mixture from Step 2 was added portionwise to the resulting mixture from Step 1 to emulsify and cooled, to which Component 11 was added to obtain a hand cream.

The hand cream thus obtained could be lightly extended on the skin, was non-sticky, and gave the users a refreshing feel. It protected skin when working with water and did not show quality change with temperature change or with time, showing superior stability.

### Example 41: Eye Liner

| Components | % |
|---|---|
| 1. M3T-C10 | 22.0 |
| 2. KF96A-6 | 5.0 |
| 3. Silicone-treated iron oxide black | 20.0 |
| 4. Vitamin E acetate | 0.2 |
| 5. Jojoba oil | 2.0 |
| 6. Bentonite | 3.0 |
| 7. KF6017 | 2.0 |
| 8. Ethanol | 10.0 |
| 9. 1,3-butylene glycol | 10.0 |
| 10. Antiseptic | q.1. |
| 11. Perfume | q.1. |
| 12. Purified water | Balance |

| | |
|---|---|
| KF96A-6 (from Shin-Etsu Chemical Co. , Ltd.) : INCI name, dimethicone with a viscosity of 6 mm ²/sec. KF6017 (from Shin-Etsu Chemical Co., Ltd.): INCI name, PEG-10 dimethicone | |

### (Preparation Method)

- Step 1:: Components 1 - 2 and 4 - 7 were mixed, and Component 3 was added to disperse homogeneously.
- Step 2:: Components 8 - 10 and 12 were mixed.
- Step 3:: The resulting mixture from Step 2 was added portionwise to the resulting mixture from Step 1 to emulsify and cooled, to which Component 11 was added to obtain an eye liner.

The eye liner thus obtained could be lightly extended on the skin, was easy to draw, had a non-sticky touch and refreshing feel. It had very good properties for use and stability, and did not show quality change with temperature change or with time. It had good water and sweat resistances as well as long lasting makeup coverage.

### Example 42: Cream

| Components | % |
|---|---|
| 1. M3T-C3 | 16.0 |
| 2. KF96A-6 | 4.0 |
| 3. KF6012 | 5.0 |
| 4. POE(5) octyldodecyl ether | 1.0 |
| 5. Polyoxyethylene sorbitan monostearate (20E.O.) | 0.5 |
| 6. SUNSPHERE SZ-5 | 2.0 |
| 7. Silicone-treated fine particle titanium dioxide | 10.0 |
| 8. Liquid paraffin | 2.0 |
| 9. Macademian nut oil | 1.0 |
| 10. Scuttellaria Root Extract | 1.0 |
| 11. Gentiana Extract | 0.5 |
| 12. Ethanol | 5.0 |
| 13. 1,3-butylene glycol | 2.0 |
| 14. Antiseptic | q.1. |
| 15. Perfume | q.1. |
| 16. Purified water | Balance |

| | |
|---|---|
| KF96A-6 (from Shin-Etsu Chemical Co., Ltd.) : ICNI name, dimethicone with a viscosity of 6 mm²/sec. KF6012 (from Shin-Etsu Chemical Co., Ltd.): ICNI name, PEG / PPG-20 / 22 butylether dimethicone). SUNSPHERE SZ-5 (from Asahi Glass Company) : Zinc oxide, Silica with a particle size ranging from 0.01 to 10µm, encapsulating 50% of anhydrous silicic acid-treated zinc oxide. Scuttellaria Root Extract: extracted with a 50% aqueous 1,3-butylene glycol solution. Gentiana Extract: extracted with a 20% aqueous ethanol solution. | |

### (Preparation Method)

- Step 1:: Components 6 - 9 were mixed to disperse homogeneously.
- Step 2:: Components 1 - 5 were mixed, to which the resulting mixture from Step 1 was added.
- Step 3:: Components 10 - 14 and Component 16 were mixed, and the resulting mixture from Step 2 was added to emulsify.
- Step 4:: After cooling the resulting mixture from Step 3, Component 15 was added to obtain a cream.

The cream thus obtained was non-sticky, could be lightly extended on the skin , showed a good affinity with skin, clung tightly to the skin, and gave a shiny finish.

It did not wear off easily and did not show quality change with temperature change or with time, showing superior stability.

### Example 43: Foundation

| Components | % |
|---|---|
| 1. M3T-C4 | 27.0 |
| 2. KF56 | 3.0 |
| 3. Glyceryl triisooctanoate | 10.0 |
| 4. KF6017 | 1.0 |
| 5. KF6105 | 1.0 |
| 5. Polyglyceryl monoisostearate | 3.0 |
| 6. Powder mixture treated for hydrophobicity¹⁾ | 18.0 |
| 7. Iron oxide red | 1.2 |
| 8. Iron oxide yellow | 2.6 |
| 9. Iron oxide black | 0.2 |
| 10. 1,3-butylene glycol | 7.0 |
| 11. Sodium chloride | 0.5 |
| 12. Antiseptic | q.1. |
| 13. Perfume | q.1. |
| 14. Purified water | Balance |

| | |
|---|---|
| 1) Mixing ratio of powder mixture treated for hydrophobicity a. Fine particle titanium dioxide 8.0 % b. Fine particle zinc oxide 4.0 % c. Talc 3.0 % d. Mica 3.0 % KF56 (from Shin-Etsu Chemical Co., Ltd.): INCI name, phenyltrimethicone KF6017 (from Shin-Etsu Chemical Co., Ltd.): INCI name, PEG-10 dimethicone KF6105 (from Shin-Etsu Chemical Co., Ltd.): INCI name, Lauryl Polyglyceryl-3 Polydimethylsiloxyethyl Dimethicone | |

### (Preparation Method)

- Stepl:: Components a - d were mixed, to which 1% of methylhydrogenpolysiloxane was added, followed by heating.
- Step 2:: Components 1 - 6 were mixed and dissolved while heating, to which Components 7 - 10 were added to disperse homogeneously.
- Step 3:: Components 11 - 13 and Component 15 were mixed, which was added to the resulting mixture from Step 2 to emulsify.
- Step 4:: After cooling the resulting mixture from Step 3, Component 14 was added to obtain a foundation.

The foundation thus obtained was non-sticky, could be lightly extended on the skin, had a good affinity with skin, clung tightly to the skin, and gave a shiny finish. It did not wear off easily and did not show quality change with temperature change or with time, showing superior stability.

### Example 44: Suncut Cream

| Components | % |
|---|---|
| 1. M3T-C6 | 17.5 |
| 2. KP545 | 12.0 |
| 3. Glycerol triisooctanoate | 5.0 |
| 4. SPD-T5 | 6.0 |
| 5. KSG210 | 5.0 |
| 6. KF6017 | 1.0 |
| 7. Zinc oxide for lipophilicity | 20.0 |
| 8. Sodium chloride | 0.5 |
| 9. 1,3-butylene glycol | 2.0 |
| 10. Antiseptic | q.1. |
| 11. Perfume | q.1. |
| 12. Purified water | Balance |

| | |
|---|---|
| KP545 (from Shin-Etsu Chemical Co., Ltd.): Acrylic silicone resin/50 % decamethylcyclopentasiloxane solution. SPD-T5 (from Shin-Etsu Chemical Co., Ltd.) : INCI name, stearic acid, cyclopentasiloxane, polyglyceryl - 3 / polydimethylsiloxyethyl dimethicone, Aluminum hydroxide, titanium oxide. KSG210 (from Shin-Etsu Chemical Co., Ltd.): INCI name, dimethicone, dimethicone / (PEG - 10 / 15) crosspolymer. KF6017 (from Shin-Etsu Chemical Co., Ltd.): INCI name, PEG-10 dimethicone. | |

### (Preparation Method)

- Step 1:: Component 2 was added to a portion of Component 1 to obtain a homogeneous mixture, to which Component 7 was added to disperse with a beads mill.
- Step 2:: The remaining portion of Component 1 and Components 3 - 6 were mixed homogeneously.
- Step 3:: Components 8 - 10 and Component 12 were mixed to dissolve.
- Step 4:: The resulting mixture from Step 3 was added to the resulting mixture from Step 2 to emulsify, to which the resulting mixture from Step 1 and Component 11 were added to obtain a suncut cream.

The suncut cream thus obtained was non-sticky, could be lightly extended on the skin, had a good affinity, clung tightly to the skin, and gave a shiny finish. The cosmetic coverage lasted long. It did not show quality change with temperature change or with time.

### Example 45: O/W Hand Cream

| Components | % |
|---|---|
| 1. KP562P | 5.0 |
| 2. M3T-C10 | 5.0 |
| 3. KSG16 | 2.0 |
| 4. Isoparaffin | 5.0 |
| 5. Vaseline | 5.0 |
| 6. Glyceryl triisooctanoate | 3.0 |
| 7. KF6017 | 0.5 |
| 8. Polyoxyethylene sorbitan monooleate | 1.0 |
| 9. Sepigel 305 | 2.0 |
| 10. 1,3-butylene glycol | 5.0 |
| 11. Glycerin | 5.0 |
| 12. Antiseptic | q.1. |
| 13. Perfume | q.1. |
| 14. Purified water | Balance |

| | |
|---|---|
| KP562P (from Shin-Etsu Chemical Co., Ltd.) : INCI name, (acrylates / behenyl acrylate / dimethicone methacrylate) copolymer. KSG16 (from Shin-Etsu Chemical Co., Ltd.): INCI name, (dimethicone / vinyldimethicone) crosspolymer. Crosslinked dimethylpolysiloxane/dimethylpolysiloxane KF6017 (from Shin-Etsu Chemical Co., Ltd.): ICNI name, PEG-10 dimethicone. Sepigel 305: (from SEPPIC Inc.) | |

### (Preparation Method)

- Step 1:: Components 1 - 7 were mixed homogeneously.
- Step 2:: Components 8 - 11 and Component 13 were mixed homogeneously.
- Step 3:: The resulting mixture from Step 2 was added to the resulting mixture from Step 1 to emulsify, to which Component 12 was added to obtain an O/W hand cream.

The hand cream thus obtained was non-sticky, could be lightly extended on the skin, had a good affinity with skin, clung tightly to the skin, and gave a shiny finish. The cosmetic coverage lasted long and it did not show quality change with temperature change or with time.

### Example 46: O/W Hand Cream

| Components | % |
|---|---|
| 1. KF7312J | 5.0 |
| 2. M3T-C3 | 5.0 |
| 3. KP561P | 8.0 |
| 4. Cetanol | 1.0 |
| 5. Glyceryl triisostearate | 5.0 |
| 6. Stearic acid | 3.0 |
| 7. Glyceryl monostearate | 1.5 |
| 8. KF6015 | 0.7 |
| 9. Sorbitan sesquioleate | 0.5 |
| 10. Polyoxyethylene sorbitan monooleate | 1.0 |
| 11. Sodium hydroxide (1% aqueous solution) | 10.0 |
| 12. 1,3-butylene glycol | 5.0 |
| 13. Antiseptic | q.1. |
| 14. Perfume | q.1. |
| 15. Purified water | Balance |

| | |
|---|---|
| KF7312J (from Shin-Etsu Chemical Co. , Ltd.) : INCI name, trimethyl siloxysilicate, cyclopentasiloxane. KF6015 (from Shin-Etsu Chemical Co., Ltd.): INCI name, PEG-3 Dimethicone. KP561P (from Shin-Etsu Chemical Co., Ltd.): INCI name, Acrylates / steary acrylate / dimethicone methacrylate) copolymer. | |

### (Preparation Method)

- Step 1:: Components 1 - 10 were mixed and dissolved while heating.
- Step 2:: Components 11 - 13 and Component 15 were mixed and heated.
- Step 3:: The resulting mixture from Step 2 was added to the resulting mixture from Step 1 to emulsify, to which Component 14 was added to obtain an O/W hand cream.

The hand cream thus obtained was non-sticky, could be lightly extended on the skin, had a high affinity with skin, clung tightly to the skin, and gave a shiny finish. It provided a long lasting cosmetic coverage and superior stability and did not Show quality change with temperature change or with time.

### Example 47: Aerosol Composition

| Components | % |
|---|---|
| 1. Silicone-treated mica | 3.0 |
| 2. Chlorohydroxyaluminum | 2.0 |
| 3. Isopropylmethylphenol | 0.3 |
| 4. Sorbitan sesquioleate | 0.2 |
| 5. Isopropyl myristate | 5.0 |
| 6. M3T-C4 | 5.0 |
| 7. Perfume | q.1. |
| 8. Propellant | Balance |

### (Preparation Method)

- Step 1: Components 1 - 7 were mixed.
- Step 2:: After putting the resulting mixture from Step 1 in an aerosol can, Component 8 was filled.

The aerosol composition of the present invention thus obtained demonstrated a high deodorizing effect, no stickiness and no tackiness in use, could be lightly extended on the skin, showed a lightweight and smooth touch. Furthermore, it demonstrated good re-dispersibility, which provided good properties for use.

### Example 48: Antiperspirant

| Components | % |
|---|---|
| 1. KSG-210 | 20.0 |
| 2. KSG-15 | 20.0 |
| 3. M3T-C6 | 30.0 |
| 4. Aluminum Zirconium Tetrachlorohydrex GLY | 20.0 |
| 5. KF-96A-(6) | 10.0 |

| | |
|---|---|
| KSG210 (from Shin-Etsu Chemical Co., Ltd.): INCI name, dimethicone, dimethicone / (PEG-10/15) crosspolymer. KSG15 (from Shin-Etsu Chemical Co., Ltd.): INCI name, (dimethicone / vinyldimethicone) crosspolymer / cyclopentasiloxane KF96A-6 (from Shin-Etsu Chemical Co., Ltd.): INCI name, Dimethicone with a viscosity of 6 mm²/sec. | |

### (Preparation Method)

- Step 1:: Components 1 - 3 and Component 5 were mixed homogeneously.
- Step 2:: Component 4 was added to the resulting mixture from Step 1 and dispersed by mixing.

The antiperspirant thus obtained was non-sticky and could be lightly extended on the skin. It did not show quality change with temperature change or with time, showing superior stability.

### Example 49: Eye Wrinkle Cream

| Components | % |
|---|---|
| 1. M3T-C10 | 20.0 |
| 2. KF7312J | 5.0 |
| 3. KF6017 | 2.0 |
| 4. KF6104 | 5.0 |
| 5. Sodium chondroitin sulfate | 2.0 |
| 6. Sodium lactate | 1.0 |
| 7. Glycerin | 50.0 |
| 8. Antiseptic | q.1. |
| 9. Antioxidant | q.1. |
| 10. Perfume | q.1. |
| 11. Purified water | Balance |

| | |
|---|---|
| KF7312J (from Shin-Etsu Chemical Co., Ltd.) : INCI name, trimethylsiloxy silicate, cyclopentasiloxane. KF6017 (from Shin-Etsu Chemical Co., Ltd.) : INCI name, PEG-10 Dimethicone KF6104 (from Shin-Etsu Chemical Co., Ltd.) : INCI name, Polyglyceryl -3 Polydimethylsiloxyethyl Dimethicone. | |

### (Preparation Method)

- Step 1:: Components 1 - 4 and Component 9 were mixed while heating.
- Step 2:: Components 5 - 8 and Component 11 were dissolved by heating.
- Step 3:: While stirring, the resulting mixture from Step 2 was added portionwise to the resulting mixture from Step 1 to emulsify and cooled, to which Component 10 was added to obtain an eye wrinkle cream.

The eye wrinkle cream thus obtained could be lightly extended on the skin, had a non-sticky, non-greasy, and lightweight touch, provided moisturized and hydrated feel to the users. It maintained long lasting cosmetic coverage. No quality change was found with temperature change or with time.

### Example 50: Makeup Remover

| Components | % |
|---|---|
| 1. POE (10) Sorbitan monolaureate | 10.0 |
| 2. M3T-C3 | 20.0 |
| 3. Sorbitol | 10.0 |
| 4. Carrageenan | 0.5 |
| 5. Glycerin | 5.0 |
| 6. Sodium citrate | 0.5 |
| 7. Antiseptic | q.1. |
| 8. Perfume | q.1. |
| 9. Purified water | 54.0 |

### (Preparation Method)

- Step 1:: Components 1 - 7 and 9 were added to dissolve homogeneously.
- Step 2:: To the resulting mixture from Step 1, Component 8 was added to obtain a makeup remover.

Upon removing foundation with the makeup remover thus obtained, it was found that the makeup remover had good compatibility with foundation, thus demonstrating high cleansing effect. It could be lightly extended in use and left skin moisturized feel. It showed good properties for use and left skin comfortable feel.

### Example 51: Hair Make Remover

| Components | % |
|---|---|
| 1. Polyoxyethylene (15) isocetyl ether¹⁾ | 10.0 |
| 2. M3T-C4 | 20.0 |
| 3. 1,3-butylene glycol | 10.0 |
| 4. Glycerin | 10.0 |
| 5. Carrageenan | 0.5 |
| 6. Sodium citrate | 0.5 |
| 7. Antiseptic | q.1. |
| 8. Perfume | q.1. |
| 9. Purified water | 49.0 |

| | |
|---|---|
| 1) Polyoxyethylene (15) Isocetyl Ether: from Sanyo Chemical Industries Co., Ltd. | |

### (Preparation Method)

- Step 1:: Components 1 - 7 and 9 were added to dissolve homogeneously.
- Step 2:: To the resulting mixture from Step 1, Component 8 was added to obtain a hair make remover.

When washing hair with the hair make remover thus obtained, it was compatible with sebum, had a good cleansing effect, could be lightly extended on the hair, and did not leave hair stickiness but left moisturized feel. It showed good properties for use and left skin comfortable feel.

### Example 52: Facial Wash

| Components | % |
|---|---|
| 1. Polyoxyethylene (6) Lauryl Ether¹⁾ | 5.0 |
| 2. M3T-C6 | 10.0 |
| 3. Ethanol | 10.0 |
| 4. Lauryldimethylamine Oxide²⁾ | 2.0 |
| 5. Propylene glycol | 3.0 |
| 6. Sodium citrate | 0.5 |
| 7. Antiseptic | 0.5 |
| 8. Perfume | q.1. |
| 9. Purified water | 69.5 |

| | |
|---|---|
| 1) Pegnol L-6 (from Toho Chemical Industry Co., Ltd.) 2) Unisafe A-LM (from Nippon Oil and Fats Co., Ltd.) | |

### (Preparation Method)

- Step 1:: Components 1 - 7 and 9 were combined and dissolved homogeneously.
- Step 2:: To the resulting mixture from Step 1, Component 8 was added to obtain a facial wash.

Upon using the facial wash thus obtained, the facial wash was found to be compatible with cosmetics and sebum and have good cleansing effect. It could be lightly extended on the skin in use, and did not leave skin stickiness but left moisturized feel. It showed good properties for use and left skin comfortable feel.

### Example 53: Makeup Remover

| Components | % |
|---|---|
| 1. POE (6) Sorbitan Monolaureate | 5.0 |
| 2. M3T-C10 | 5.0 |
| 3. KF6105 | 15.0 |
| 4. Ethanol | 10.0 |
| 5. Glycerin | 2.0 |
| 6. Dipropylene Glycol | 3.0 |
| 7. Sodium Glutamate | 0.5 |
| 8. Antiseptic | q.1. |
| 9. Perfume | q.1. |
| 10. Purified water | 59.5 |

| | |
|---|---|
| KF6105 (from Shin-Etsu Chemical Co., Ltd.): INCI name, Lauryl Polyglyceryl-3 Polydimethylsiloxyethyl Dimethicone | |

### (Preparation Method)

- Step 1:: Components 1 - 8 and 10 were combined and dissolved homogeneously.
- Step 2:: To the resulting mixture from Step 1, Component 9 was added to obtain a makeup remover.

Upon using the makeup remover thus obtained, the makeup remover was found to be compatible with cosmetics and sebum and have good cleansing effect. It could be lightly extended on the skin in use, and did not leave skin stickiness but left moisturized feel. It showed good properties for use and left skin comfortable feel.

### Example 54: Polyalcohol-in-Oil Emulsified Cosmetic

| Components | % |
|---|---|
| 1. KSG15 | 30.0 |
| 2. M3T-C3 | 15.0 |
| 3. Dimethylpolysiloxane (6 mm² / sec at 25 degrees C) | 7.0 |
| 4. KF6104 | 3.0 |
| 5. Dimethyldistearylammonium hectorite | 2.0 |
| 6. Antiseptic | q.1. |
| 7. Perfume | q.1. |
| 8. Sodium chloride | 0.05 |
| 9. 1,3-butylene glycol | 42.95 |

| | |
|---|---|
| KSG15 (from Shin-Etsu Chemical Industries Co., Ltd.); INCI name, cyclopentasiloxane, (dimethicone / vinyldimethicone) crosspolymer. KF6104 (from Shin-Etsu Chemical Co., Ltd.); INCI name, Polyglyceryl-3 Polydimethylsiloxyethyl Dimethicone | |

### (Preparation Method)

- Step 1:: Components 1 - 5 and 7 were mixed homogeneously.
- Step 2:: Components 6, 8, and 9 were mixed.
- Step 3:: The resulting mixture from Step 2 was added to the resulting mixture from Step 1 to emulsify homogeneously.

The polyalcohol-in-oil emulsified cosmetic thus obtained was stable, could be lightly extended on the skin, had a non-sticky and non-greasy touch, and left skin moisturized feel.

### Example 55: Solid Emulsified Blush of Polyalcohol-in-Oil Type

| Components | % |
|---|---|
| 1. KSG15 | 5.0 |
| 2. Decamethylcyclopentasiloxane | 5.0 |
| 3. M3T-C4 | 19.7 |
| 4. Cetyl Isooctanoate | 15.0 |
| 5. Paraffin wax (mp; 80 degrees C) | 12.0 |
| 6. KF6104 | 3.0 |
| 7. Dimethyldisearylammonium hectorite | 0.2 |
| 8. Powder treated for hydrophobicity | 25.0 |
| 9. Sodium citrate | 0.1 |
| 10. Antiseptic | q.1. |
| 11. Perfume | q.1. |
| 12. 1,3-Dibutylene glycol | 15.0 |

| | |
|---|---|
| KSG15 (from Shin-Etsu Chemical Co., Ltd.) (from Shin-Etsu Chemical Co., Ltd.): INCI name, (dimethicone / vinyldimethicone) crosspolymer / cyclopentasiloxane. KF6104 (from Shin-Etsu Co., Ltd.): INCI name, Polyglyceryl-3 Polydimethylsiloxyethyl Dimethicone. | |

### (Preparation Method)

- Step 1:: Components 1 -7 and 11 were heated to 80 degrees C to mix homogeneously.
- Step 2:: Component 8 was added to the resulting mixture from Step 1 and dispersed homogeneously.
- Step 3:: Components 9, 10, and 12 were heated to 80 degrees C and the resulting mixture was added to the mixture from Step 2 to pour into a metal plate to cool down.

The solid emulsified blush of polyalcohol-in-oil was stable, could be lightly extended on the skin, had a non-sticky and non-greasy touch, and left skin moisturized feel.

### Example 56: Creamy Lipstick

| Components | % |
|---|---|
| 1. Palmitic acid / dextrin ethylhexanoate ¹⁾ | 9.0 |
| 2. Glyceryl triisooctanoate | 22.0 |
| 3. Bentonite | 0.7 |
| 4. KF6104 | 1.5 |
| 5. M3T-C6 | 42.0 |
| 6. 1,3-butylene glycol | 5.0 |
| 7. Sodium chloride | 0.5 |
| 8. Purified water | 19.3 |
| 9. Coloring pigment | q.1. |

| | |
|---|---|
| 1) Rheopal TT (from Chiba Seifun Co., Ltd.) | |

### (Preparation Method)

- Step 1:: Portions of Components 1 and 2 and Components 3 - 5 were mixed to dissolve.
- Step 2:: To the rest of Component 2, Component 9 was added to disperse with rollers.
- Step 3:: To the resulting mixture from Step 2, the mixture from Step 1 was added and mixed homogeneously.
- Step 4:: Components 6 - 8 were mixed while heating.
- Step 5:: The resulting mixture from Step 4 was added to the mixture from Step 3 to emulsify.

The lipstick thus obtained was a long wearing creamy lipstick of w/O type, could be lightly extended, had a non-sticky and non-greasy touch.

### Example 57: Suntan Milky Lotion

| Components | % |
|---|---|
| 1. Emulsifier composition ¹⁾ | 6.0 |
| 2. M3T-C10 | 49.0 |
| 3. 1,3-butylene glycol | 5.0 |
| 4. Sodium dehydroacetate | 0.2 |
| 5. Antioxidant | q.1. |
| 6. Antiseptic | q.1. |
| 7. Perfume | q.1. |
| 8. Purified water | 39.8 |

| | |
|---|---|
| 1) The mixing ratio of the emulsifier composition: a. KF6017 (from Shin-Etsu Co., Ltd.); ICNI name, PEG-10 dimethicone: 10 parts by weight b. Dioctadecyldimethylammonium-modified montmorillonite: 10.0 parts by weight. c. Ethanol: 40.0 parts by weight | |

### (Preparation Method)

- Step 1: Component a was dissolved in Component c and Component b was added.
- Step 2: The resulting mixture from Step 1 was stirred for an hour with a disperser and then ethanol was evaporated with a evaporator.
- Step 3: The resulting mixture from Step 2 was dried overnight at 50 degrees C to obtain an emulsified composition, Component 1.
- Step 4: Component 1, obtained in Step 3, was mixed with Component 2.
- Step 5: Components 3 - 6 and 8 were mixed homogeneously.
- Step 6: Under stirring, the resulting mixture from Step 4 was added portionwise to the resulting mixture from Step 5 to emulsify and then Component 7 was added to obtain a suntan milky lotion.

The suntan milky lotion thus obtained could be lightly extended on the skin, had a fine texture and a non-sticky and non-greasy touch, left skin moisturized, hydrated and refreshed feel to the users, was water-proof and the coverage wore long. It did not also show quality change with temperature change or with time, showing superior stability.

### Example 58: Suncut Cream

| Components | % |
|---|---|
| 1. M3T-C3 | 17.5 |
| 2. Acrylic silicone resin / decamethylcyclopentasiloxane ¹⁾ | 12.0 |
| 3. Glyceryl triisooctanoate | 5.0 |
| 4. Octyl para-methoxycinnamate | 6.0 |
| 5. Cross-linked polyether-modified silicone ²⁾ | 5.0 |
| 6. KF6104 | 1.0 |
| 7. Zinc oxide treated for lipophilicity | 20.0 |
| 8. Sodium chloride | 0.5 |
| 9. 1,3-butylene glycol | 2.0 |
| 10. Antiseptic | q.1. |
| 11. Perfume | q.1. |
| 12. Purified water | 31.0 |

| | |
|---|---|
| 1) Acrylic silicone / decamethylcyclopentasiloxane: KP545 (from Shin-Etsu Co., Ltd.) 2)Cross-linked polyether-modified silicone: KSG21 (from Shin-Etsu Co., Ltd.) KF6104 (from Shin-Etsu Co., Ltd.): INCI name, Polyglyceryl-3 Polydimethylsiloxyethyl Dimethicone. | |

### (Preparation Method)

- Step 1:: To a portion of Component 1, Component 2 was added and mixed homogeneously, to which Component 7 was added and dispersed with a beads mill.
- Step 2:: The rest of Component 1 and Components 3 - 6 were combined to mix homogeneously.
- Step 3:: Component 8 - 10 and 12 were mixed to dissolve homogeneously.
- Step 4:: To the resulting mixture from Step 2, the mixture from Step 3 was added and emulsified, to which the mixture from Step 1 and Component 11 were added to obtain a suncut cream.

The suncut cream thus obtained could be lightly extended on the skin, had a non-sticky touch, demonstrated good affinity with skin, clung tightly to the skin, and gave a shiny finish. It also exhibited a long lasting coverage and no quality change was observed with temperature change or with time.

### Example 59: Suncut Milky Lotion

| Components | % |
|---|---|
| 1. M3T-C4 | 3.0 |
| 2. Dimethylpolysiloxane with a viscosity of 6 mm² / sec) | 5.0 |
| 3. Glyceryl triisooctanoate | 5.0 |
| 4. KF6105 | 1.0 |
| 5. Cross-linked polyether-modified silicone ¹⁾ | 3.0 |
| 6. Titanium oxide / decamethylcyclopentasiloxane dispersion ²⁾ | 25.0 |
| 7. Zinc oxide / decamethylcyclopentasiloxane dispersion ³⁾ | 35.0 |
| 8. Dipropyleneglycol | 3.0 |
| 9. Sodium citrate | 0.5 |
| 10. Antiseptic | q.1. |
| 11. Perfume | q.1. |
| 12. Purified water | 19.5 |

| | |
|---|---|
| KF6105 (from Shin-Etsu Co., Ltd.):INCI name, Lauryl Polyglyceryl-3 Polydimethylsiloxyethyl Dimethicone 1) KSG-21 (from Shin-Etsu Co., Ltd.) 2) SPD-T1S (from Shin-Etsu Co., Ltd.) 3) SPD-Z1S (from Shin-Etsu Co., Ltd.) | |

### (Preparation Method)

- Step 1: Components 1 - 5 were combined to mix homogeneously.
- Step 2: Components 8, 10 and 12 were combined to dissolve.
- Step 3: To the resulting mixture from Step 1, the mixture from Step 2 was added, and then Component 6, 7 and 11 were added to obtain a suncut milky lotion.

The suncut milky lotion could be lightly extended on the skin, had a non-sticky touch, exhibited a good affinity with skin, clung tightly to the skin, and gave a shiny finish. The coverage lasted long and no quality change was observed with temperature change or with time.

### Example 60: Hair Cream

| Components | % |
|---|---|
| 1. Dissolved silicone gum (400,000 mPa sec) | 18.0 |
| 2. Silicone network resin ¹⁾ | 6.0 |
| 3. Glyceryl tri-2-ethylhexenoic acid | 8.0 |
| 4. Vaseline | 5.0 |
| 5. Stearyl alcohol | 2.0 |
| 6. Sorbitan monoleaate | 2.0 |
| 7. KF6100 | 2.0 |
| 8. Glycerol | 5.0 |
| 9. Sodium chloride | 0.5 |
| 10. Perfume | q.1. |
| 11. Purified water | 51.5 |

| | |
|---|---|
| Dissolved silicone gum: a dissolved product of dimethicone with a viscosity of 30, 000, 000 mm² / sec (20 %) and M3T-C3 (80%). 1) A 50 % solution of network the silicone compound with a ratio of [Me₃SiO_{1/2}]/[SiO₂] of 0.8 in D5. KF6100 (from Shin-Etsu Co., Ltd.): INCI name, Polyglyceryl-3 disiloxane dimethicone | |

### (Preparation Method)

- Step 1: Components 1 -7 were mixed while heating.
- Step 2: Components 8 - 9 and 11 were mixed and stirred.
- Step 3: While stirring, to the resulting mixture from Step 2, the resulting mixture from Step 1 was added portionwise to emulsify, to which Component 10 was added to obtain a hair cream.

The hair cream thus obtained could be lightly extended on the skin, had a non-sticky and non-greasy touch, left moisturized, hydrated, and refreshed feel, and gave hair shiny gloss and smoothness. It was also found that it had a good setting effect for hair.

### Example 61: Moisturizing Cream

| Components | % |
|---|---|
| 1. Decamethylcyclopentasiloxane | 10.0 |
| 2. M3T-C6 | 3.0 |
| 3. Liquid paraffin | 5.0 |
| 4. Stearoxy-modified silicone ¹⁾ | 8.0 |
| 5. KF6009 | 2.0 |
| 6. Spherical organopolysilxane elastomer powder ²⁾ | 2.5 |
| 7. Silica treated for hydrophobicity ³⁾ | 2.0 |
| 8. Zinc Stearate | 2.0 |
| 9. Vitamin E acetate | 3.0 |
| 10. Polyethylene glycol 400 | 1.0 |
| 11. Sodium lactate | 1.0 |
| 12. 1,3-butylene glycol | 5.0 |
| 13. Antiseptic | q.1. |
| 14. Perfume | q.1. |
| 15. Purified water | 55.5 |

| | |
|---|---|
| KF6009 (from Shin-Etsu Co., Ltd.): PEG-9 Dimethicone (silicone modified with polyether at both ends) 1) KF-7002 (from Shin-Etsu Co., Ltd.) 2) KMP-590 (from Shin-Etsu Co., Ltd.) 3) Aerosil R972 (from Nippon Aerosil Corp.) | |

### (Preparation Method)

- Step 1: Components 1 - 5, 8, and 9 were mixed homogeneously, to which the Components 6 - 7 were added and dispersed homogeneously.
- Step 2: Components 10 - 13 and 15 were combined to dissolve.
- Step 3: The resulting mixture from Step 2 was added portionwise to that from Step 1 and cooled, to which Component 14 was added to obtain a moisturizing cream.

The moisturizing cream thus obtained could be lightly extended on the skin, had a non-sticky touch and gave skin moisturized and hydrated feel. It did not show quality change with temperature change or with time, showing good stability.

### Example 62: Antiperspirant

| Components | % |
|---|---|
| 1. M3T-C3 | 30.0 |
| 2. KF6017 | 1.0 |
| 3. Polyoxyethylenesorbitan monooleate (20 E.O.) | 0.5 |
| 4. Aluminum zirconium tetrachlorohydrate glycine salt | 20.0 |
| 5. Purified water | 48.5 |

| | |
|---|---|
| KF6017 (from Shin-Etsu Chemical Co., Ltd.): INCI name PPG-10 dimethicone. | |

### (Preparation Method)

- Step 1: Components 1 and 2 were mixed.
- Step 2: Component 4 was dissolved in Component 5, and Component 3 was added.
- Step 3:: While stirring, the resulting mixture from Step 2 was added portionwise to the resulting mixture from Step 1 to emulsify to obtain an antiperspirant.

The antiperspirant thus obtained could be lightly extended on the skin, had a non-sticky and non-greasy touch, and did not leave too much white powdery residue, and gave a refreshed feel to the users. It did not show quality change with temperature change or with time, showing superior stability.

### Example 63: Cleansing Cream

| Components | % |
|---|---|
| 1. M3T-C10 | 5.0 |
| 2. Methylphenylpolysiloxane | 5.0 |
| 3. Liquid paraffin | 8.0 |
| 4. Jojoba oil | 2.0 |
| 5. KF6105 | 2.5 |
| 6. KF6017 | 0.5 |
| 7. Dextrin fatty acid ester | 0.8 |
| 8. Aluminum monostearate | 0.2 |
| 9. Aluminum chloride | 1.0 |
| 10. Glycerin | 10.0 |
| 11. Antiseptic | q.1. |
| 12. Perfume | q.1. |
| 13. Purified water | 65.0 |

| | |
|---|---|
| KF6105 (from Shin-Etsu Co., Ltd.): INCI name, Lauryl Polyglyceryl-3 Polydimethylsiloxyethyl Dimethicone KF6017 (from Shin-Etsu Co., Ltd.): INCI name, PEG-10 dimethicone | |

### (Preparation Method)

- Step 1: Components 1 - 8 were blended while heating.
- Step 2: Components 9 - 11 and 13 were dissolved while heating.
- Step 3: While stirring, the resulting mixture from the Step 2 was added portionwise to that from Step 1 and cooled, to which Component 12 was added to obtain a cleansing cream.

The cleansing cream thus obtained could be lightly extended on the skin, had a fine texture and a non-sticky and non-greasy touch, and left skin moisturized, hydrated and refreshed feel. It also had high cleansing effect and did not show a quality change with temperature change and with time, showing superior stability.

### Example 64 Treatment Gel

| Components | % |
|---|---|
| 1. M3T-C3 | 5.0 |
| 2. Ethanol | 15.0 |
| 3. KF6018 | 0.5 |
| 4. Glyceryl triisooctanoate | 3.0 |
| 5. Stearoxy-modified silicone ¹⁾ | 2.0 |
| 6. Silicone composite powder ²⁾ | 8.0 |
| 7. Carboxy vinyl polymer (1% aqueous solution) | 20.0 |
| 8. Triethanol amine | 0.2 |
| 9. Antiseptic | q.1. |
| 10. Perfume | q.1. |
| 11. Purified water | 46.3 |

| | |
|---|---|
| KF6018 (from Shin-Etsu Chemical Co., Ltd.) : INCI name, PEG-11 methyletherdimethicone 1) KF-7002 (from Shin-Etsu Chemical Co., Ltd.) 2) KSP-100 (from Shin-Etsu Chemical Co., Ltd.) | |

### (Preparation Method)

- Step 1: Components 1 - 6 were combined and dispersed.
- Step 2: Components 7 - 9 and 11 were mixed to obtain a homogeneous mixture.
- Step 3: The resulting mixture from the Step 1 was added to that from the Step 2, to which Component 8 was added to mix homogeneously.

The treatment gel thus obtained could be lightly extended on the skin, had a non-sticky and non-greasy touch, and left skin moisturized, hydrated and refreshed feel. It also had high affinity with skin and did not show quality change with temperature change or with time, showing superior stability.

### Example 65: Wash-off Pack

| Components | % |
|---|---|
| 1. M3T-C3 | 3.0 |
| 2. KF6018 | 2.0 |
| 3. Kaolin | 30.0 |
| 4. Carboxy vinylpolymer | 0.4 |
| 5. 1,3-butylene glycol | 10.0 |
| 6. Glycerin | 20.0 |
| 7. Triethanolamine | 0.4 |
| 8. Antiseptic | q.1. |
| 9. Perfume | q.1. |
| 10. Purified water | 34.2 |

| | |
|---|---|
| KF6018 (from Shin-Etsu Co., Ltd.): INCI name, PEG-11 methyl ether dimethicone | |

### (Preparation Method)

- Step 1: Components 1, 2 and 8 were mixed.
- Step 2: Components 4 - 7 and 10 were mixed homogeneously and then Component 3 was added to mix.
- Step 3: To the resulting mixture from Step 2, that from Step 1 was added to emulsify, to which Component 9 was added to obtain a paste of wash-off pack.

The wash-off pack thus obtained could be lightly extended on the skin, was highly effective in washing the skin, and left skin moisturized, non-sticky, and smooth feels after washing off. It had good properties for use and superior stability.

### Example 66: O / W / O Milky Lotion

| Components | % |
|---|---|
| 1. Cross-linked polyether-modified silicone ¹⁾ | 3.0 |
| 2. KF6104 | 1.0 |
| 3. Gryceryl trioctanoate | 14.0 |
| 4. Cross-linked alkyl-modified silicone compound ²⁾ | 5.0 |
| 5. Sucrose monostearate | 3.0 |
| 6. Glycerin | 5.0 |
| 7. 1,3-butylene glycol | 5.0 |
| 8. Antiseptic | q.1. |
| 9. Purified water | 60.0 |
| 10. Macadamia nut oil | 2.0 |
| 11. Cetyl alcohol | 2.0 |
| 12. Perfume | q.1. |

| | |
|---|---|
| KF6104 (from Shin-Etsu Co., Ltd.): INCI name, Polyglyceryl-3 Polydimethylsiloxyethyl Dimethicone 1) A paste composition comprising 25 % of cross-linked polyether-modified silicone and 75 % of M3T-C3. 2) KSG-43 (from Shin-Etsu Co., Ltd.) | |

### (Preparation Method)

- Step 1: Components 1 - 4 were mixed homogeneously.
- Step 2: Components 5 - 9 were mixed while heating to obtain a homogeneous mixture.
- Step 3: Components 10 -12 were mixed while heating.
- Step 4: The resulting mixture from Step 3 was added to that from Step 2 while stirring to emulsify and cool.
- Step 5: The resulting mixture from Step 4 was added to that from Step 1 while stirring to emulsify.

The milky lotion thus obtained could be lightly extended on the skin, had a non-sticky and non-greasy touch, and gave a transparent finish. The coverage wore long and it did not show quality change with temperature change or with time. It was a superior O / W / O type milky lotion with good properties for use and stability.

### Example 67: O / W / O Type Liquid Foundation

| Components | % |
|---|---|
| 1. Cross-linked polyether-modified silicone¹⁾ | 4.0 |
| 2. KF6104 | 1.0 |
| 3. Propylene glycol decanoate | 5.0 |
| 4. Isopropyl myristate | 5.0 |
| 5. Pigment | 10.0 |
| 6. Hydrogenated phospholipid from egg yolk | 1.0 |
| 7. Glycerin | 2.0 |
| 8. 1,3-butylene glycol | 10.0 |
| 9. Antiseptic | q.1. |
| 10. Purified water | 52.0 |
| 11. Squalane | 5.0 |
| 12. Cetyl alcohol | 5.0 |
| 13. Perfume | q.1. |

| | |
|---|---|
| 1) A paste composition comprising 25 % of cross-linked polyether-modified silicone and 75 % of M3T-C3 2) KF6104 (from Shin-Etsu Co., Ltd.): INCI name, Polyglyceryl-3 Polydimethylsiloxyethyl Dimethicone | |

### (Preparation Method)

- Step 1: Components 1 -4 were mixed homogeneously.
- Step 2: Components 5 - 10 were mixed while heating to obtain a homogeneous mixture.
- Step 3: Components 11 - 13 were mixed while heating.
- Step 4: The resulting mixture from Step 3 was added to that from Step 2 while stirring to emulsify and cool.
- Step 5: The resulting mixture from Step 4 was added to that from Step 1 while stirring to emulsify.

The liquid foundation thus obtained could be lightly extended on the skin, had a non-sticky and non-greasy touch and gave a transparent finish. It gave a long lasting coverage and no quality change was observed with temperature change and with time. It was a superior O / W / O type liquid foundation with good properties for use and stability.

### Example 68: Oil-based Foundation

| Components | % |
|---|---|
| 1. Cellulose fatty acid ester | 6.0 |
| 2. Ceresine | 7.0 |
| 3. Polybutene | 4.0 |
| 4. Liquid paraffin | 20.0 |
| 5. M3T-C6 | 14.0 |
| 6. KF6105 | 6.0 |
| 7. Titanium oxide | 33.0 |
| 8. Tintanated mica | 3.0 |
| 9. Pigment | 7.0 |
| 10. Antiseptic | q.1. |
| 11. Perfume | q.1. |

| | |
|---|---|
| KF6105 (from Shin-Etsu Co., Ltd.): INCI name, Lauryl Polyglyceryl-3 Polydimethylsiloxyethyl Dimethicone | |

### (Preparation Method)

- Step 1: Components 1 - 6 were dissolved while heating.
- Step 2: Components 7 - 12 were mixed with the resulting mixture from Step 1.
- Step 3: The resulting mixture from Step 2 was dispersed homogeneously with a three-roller mill.
- Step 4: The resulting mixture from Step 3 was dissolved while heating, degassed, and then poured into a metal plate, followed by cooling.

The foundation thus obtained could be lightly extended on the skin, had a non-sticky and non-greasy touch, and gave a transparent finish. It also gave a long lasting coverage and no quality change was observed with temperature change or with time.

### Example 69: Lipstick

| Components | % |
|---|---|
| 1. Micro crystalline wax | 6.0 |
| 2. Synthetic hydrocarbon wax | 8.0 |
| 3. Serecine wax | 5.0 |
| 4. Candellila wax | 2.0 |
| 5. Pentaerythritol rosinate | 5.0 |
| 6. Cetyl 2-ethylhexanoate | 20.0 |
| 7. Glyceryl trioctanoate | 25.0 |
| 8. KF6105 | 5.0 |
| 9. M3T-C10 | 5.0 |
| 10. Pigment | 5.0 |
| 11. Titanated mica | 15.0 |
| 12. Perfume | q.1. |

| | |
|---|---|
| KF6105 (from Shin-Etsu Co., Ltd.); INCI name, Lauryl Polyglyceryl-3 Polydimethylsiloxyethyl Dimethicone | |

### (Preparation Method)

- Step 1: Components 1 -11 were dissolved while heating.
- Step 2: After degassing, Component 14 was added and the resulting mixture was poured into a container.

The lipstick thus obtained had a product surface with a shiny gloss and did not give sticky feel after wearing. It also had a good affinity with skin but did not cause color transferring, color fading, or color blurring. It was a high quality lipstick with a bright coloration.

### Example 70: Lipstick

| Components | % |
|---|---|
| 1. Candelilla wax | 8.0 |
| 2. Polyethylene wax | 8.0 |
| 3. Acrylic silicone resin containing long alkyl chain¹⁾ | 12.0 |
| 4. M3T-C6 | 3.0 |
| 5. Isotridecyl isononanate | 20.0 |
| 6. Glyceryl isostearate | 16.0 |
| 7. KF6105 | 0.5 |
| 8. Octadecyldimethylbenzyl ammonium-modified montmorillonite | 0.5 |
| 9. Polyglyceryl triisostearate | 27.5 |
| 10. Red No. 202 treated with graft copolymer of acrylic - silicone type²⁾ | 0.8 |
| 11. Iron oxide red treated with graft copolymer of acrylic - silicone type²⁾ | 1.5 |
| 12. Iron oxide yellow treated with graft copolymer of acrylic - silicone type²⁾ | 1.0 |
| 13. Iron oxide black treated with graft copolymer of acrylic - silicone type²⁾ | 0.2 |
| 14. Titanium oxide treated with graft copolymer of acrylic - silicone type²⁾ | 1.0 |
| 15. Antiseptic | q.1. |
| 16. Perfume | q.1. |

| | |
|---|---|
| KF6105 (from Shin-Etsu Co., Ltd.): INCI name, Lauryl Polyglyceryl-3 Polydimethylsiloxyethyl Dimethicone 1) KP-561 (from Shin-Etsu Co., Ltd.) 2) KP-541 (from Shin-Etsu Co., Ltd.) | |

### (Preparation)

- Step 1: Components 1 - 8 and a portion of Component 9 were mixed while heating to dissolve.
- Step 2: Components 10 - 16 and the rest of Component 9 were mixed homogeneously, and then the resulting mixture was added to the mixture from Step 1 to obtain a homogeneous mixture.

The lipstick thus obtained had a shiny gloss on its surface, glided on lightly, had a non-sticky and non-powdery touch, and left skin refreshed and lightweight feel. It had good water resistance and water repellency and the long lasting coverage and exhibited good stability.

### Example 71: Brushing Spray

| Components | % |
|---|---|
| 1. M3T-C3 | 5.0 |
| 2. Stearyltrimethylammonium chloride | 0.05 |
| 3. KF6105 | 0.5 |
| 4. Aluminum magnesium silicate | 0.1 |
| 5. Zinc oxide treated with oil | 3.0 |
| 6. Ethanol | 25.0 |
| 7. Perfume | q.1. |
| 8. Propellent | Balance |

### (Preparation Method)

- Step 1: Components 1 - 7 were mixed.
- Step 2: The resulting mixture from Step 1 was put in an aerosol can, to which Component 8 was put to obtain a brushing agent.

The brushing spray thus obtained gave a shiny and smooth finish and the coverage lasted long. It also demonstrated a good dispersibility of powder when using and made hair shiny and easy to comb.

### Example 72: Roll-on Antiperspirant

| Components | % |
|---|---|
| 1. KSG-210 | 20.0 |
| 2. M3T-C4 | 10.0 |
| 3. KSG-15 | 14.3 |
| 4. Decamethylcyclopentasiloxane | 30.0 |
| 5. KF6105 | 0.5 |
| 6. Organo-modified Bentonite | 0.2 |
| 7. Aluminum Zirconium Tetrachlorohydrate | 20.0 |
| 8. Zinc oxide treated with dimethylmethylhydrogen | 5.0 |
| 9. Perfume | q.1. |

| | |
|---|---|
| KSG-210 (from Shin-Etsu Co., Ltd.); INCI name, Dimethicone, Dimethicone / (PEG-10 / 15) Crosspolymer. KSG-15 (from Shin-Etsu Co., Ltd.); INCI name, Cyclopentasiloxane, (Dimethicone / Vinyl Dimethicone) Crosspolymer KF6105 (from Shin-Etsu Co., Ltd.); INCI name, Lauryl Polyglyceryl-3 Polydimethylsiloxyethyl Dimethicone | |

### (Preparation Method)

- Step 1: Components 1 -6 were mixed.
- Step 2: To the resulting mixture from Step 1, Components 7 - 9 were added to disperse homogeneously.

The roll-on antiperspirant could be lightly extended on the skin, gave a refreshing feel, and had a non-sticky and non-greasy touch. It did not show quality change with temperature change and with time, showing good properties for use and stability.

### Industrial Applicability

The cosmetics of the present invention comprise a specific silicone compound so that they do not cause dry feel. The cosmetics of the present invention also provide long lasting makeup coverage as well as superior stability at low temperature.

## Claims

1. A cosmetic composition comprising
5 to 50 mass % of a silicone compound (a) represented by the following general formula (1),
{(CH₃)₃SiO}₃SiR¹ (1)
wherein R¹ represents a monovalent alkyl group having 2 to 20 carbon atoms,
0.1 to 20 mass % of at least one nonionic surfactant selected from the group consisting of polyoxyalkylene-modified organopolysiloxane, polyglycerin-modified organopolysiloxane, and polyoxyalkylene / alkyl co-modified-organopolysiloxane, and water.

2. The cosmetic composition according to claim 1, wherein the nonionic surfactant is polyoxyalkylene-modified organopolysiloxane.

3. The cosmetic composition according to Claim 1 or 2, which further comprises an oil agent (b).

4. The cosmetic composition according to Claim 3, wherein at least a part of said oil agent (b) is liquid at 25 degrees C.

5. The cosmetic composition according to Claim 3 or 4, wherein at least a part of the oil agent (b) is a silicone compound other than the silicone compounds represented by the formula (1).

6. The cosmetic composition according to any one of Claims 3 to 5, wherein at least a part of the oil agent (b) is one silicone compound selected from the group consisting of linear organopolysiloxanes represented by the following general formula (2), cyclic organopolysiloxanes represented by the general formula (3), and branched organopolysiloxanes represented by the general formula (4): 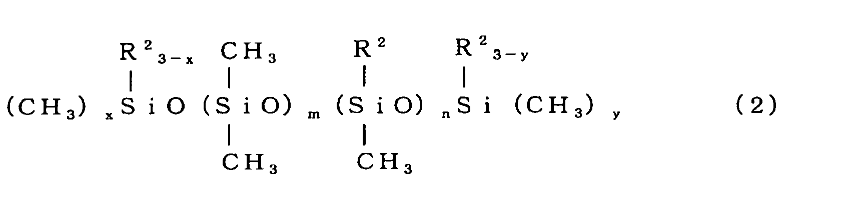 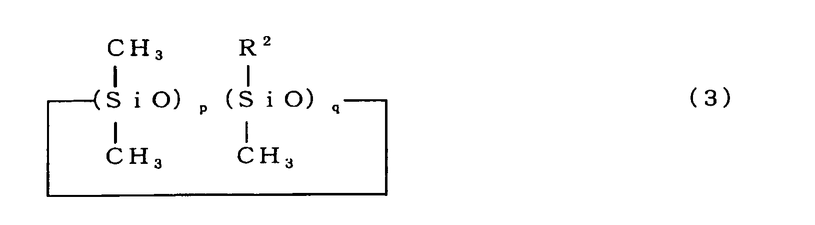
(CH₃)₄₋ᵣSi{OSi(CH₃)₃}ᵣ (4)
wherein R² represents, independently of each other, a group selected from the group consisting of a hydrogen atom, hydroxyl group, monovalent unsubstituted or fluorine-substituted alkyl groups, aryl groups, amino-substituted alkyl groups, alkoxy groups having 2 to 20 carbon atoms, and a group represented by the general formula: (CH₃)₃SiO{(CH₃)₂SiO}s Si(CH₃)₂CH₂CH₂-, m is an integer of from 0 to 1,000, n is an integer of from 0 to 1,000, m + n is an integer of from 1 to 2,000, x and y are each 0,1, 2, or 3, p and q are each an integer of from 0 to 8 with 3 ≤ p + q ≤ 8, r is an integer of from 1 to 4, and s is an integer of from 0 to 500.

7. A cosmetic composition according to Claim 6, wherein at least a part of the oil agent is selected from the group consisting of the linear organopolysiloxanes of the general formula (2), wherein at least a part of R² is an amino-substituted or fluorine substituted alkyl group having 2-20 carbon atoms, a cyclic organopolysiloxane represented by the general formula (3) containing a -{(CF₃CH₂CH₂)(CH₃)SiO}_{q-} unit, perfluoropolyether, perfluorodecalin, and perfluorooctane.

8. The cosmetic composition according to any one of Claims 1 to 7, which further comprises a compound having an alcoholic hydroxyl group (c) other than higher alcohols.

9. The cosmetic composition according to Claim 8, wherein the compound (c) is a water-soluble mono- or poly-valent alcohol having 2 to 10 carbon atoms.

10. The cosmetic composition according to any one of Claims 1 to 9, which further comprises a water-soluble polymer and/or a water-swelling polymer (d).

11. The cosmetic composition according to any one of Claims 1 to 10, which further comprises powder and/or a coloring agent.

12. The cosmetic composition according to Claim 11, wherein at least a part of the powder and/or coloring agent is selected from the group consisting of spherical silicone elastomer powder, spherical polymethylsilsesquioxane powder, spherical silicone elastomer powder with its surface coated with polymethylsilsesquioxane, polyethylene powder, polypropylene powder, polytetrafluoroethylene powder, and polyurethane powder.

13. The cosmetic composition according to any one of Claims 1 to 12, which further comprises a surfactant (g).

14. The cosmetic composition according to any one of Claims 1 to 13, which further comprises a cross-linked organopolysiloxane (h).

15. The cosmetic composition according to Claim 14, wherein the cross-linked organopolysiloxane (h) is in a swelled form where it is swelled with a larger amount in weight of silicone having a viscosity of 0.65 mm²/sec to 100 mm²/sec than the amount of the organopolysiloxane (h).

16. The cosmetic composition according to Claim 14 or 15, wherein the cross-linked organopolysiloxane (h) is one obtained by reacting an organopolysiloxane having two or more vinylic reactive sites with an organohydrogenpolysiloxane having a hydrogen atom which is bonded to a silicon atom.

17. The cosmetic composition according to any one of Claims 14 to 16, wherein the cross-linked organopolysiloxane (h) has at least one residue selected from the group consisting of polyoxyalkylene residues, alkyl residues, aryl residues, polyglycerin residues and fluoroalkyl residues.

18. The cosmetic composition according to any one of Claims 1 to 17, which further comprises a silicone resin (i) which is gummy or solid at 25 degrees C.

19. The cosmetic composition according to Claim 18, wherein the silicone resin (i) is at least one network silicone compound represented by MQ, MT, MDQ, MDT, MTQ, MDTQ, TD, TQ, or TDQ.

20. The cosmetic composition according to Claim 18 or 19, wherein the silicone resin (i) is a network silicone compound having at least one residue selected from the group consisting of pyrrolidone residues, long chain alkyl residues, polyoxyalkylene residues, fluoroalkyl residues, and amino residue.

21. The cosmetic composition according to any one of Claims 18 to 20, wherein the silicone resin (i) is an acrylic silicone resin.

22. The cosmetic composition according to any one of Claims 1 to 21, which further comprises a UV protecting component (j).

23. The cosmetic composition according to any one of Claims 1 to 22, wherein the cosmetic composition is a skin care cosmetic, a hair care cosmetic, an antiperspirant, a makeup cosmetic, or a UV protecting cosmetic.

24. The cosmetic composition according to any one of Claims 1 to 23, wherein the cosmetic composition is in a form of liquid, emulsion, cream, solid, paste, gel, powder, multi layers, mousse, or spray.

## Patentansprüche

1. Kosmetische Zusammensetzung, umfassend 5 bis 50 Gew.-% einer Siliconverbindung (a), verkörpert durch die folgende allgemeine Formel (I)
{ (CH₃)₃SiO}₃SiR¹ (1)
worin R¹ eine einwertige Alkylgruppe mit 2 bis 20 Kohlenstoffatomen verkörpert, 0,1 bis 20 Gew.-% mindestens eines nichtionischen Tensids, ausgewählt aus der Gruppe, bestehend aus Polyoxyalkylen-modifiziertem Organopolysiloxan, Polyglycerin-modifiziertem Organopolysiloxan und Polyoxyalkylen-/ Alkyl-co-modifiziertem Organopolysiloxan, und Wasser.

2. Kosmetische Zusammensetzung gemäß Anspruch 1, wobei das nichtionische Tensid Polyoxyalkylen-modifiziertes Organopolysiloxan ist.

3. Kosmetische Zusammensetzung gemäß Anspruch 1 oder 2, die des Weiteren ein Ölmittel (b) umfasst.

4. Kosmetische Zusammensetzung gemäß Anspruch 3, wobei mindestens ein Teil des genannten Ölmittels (b) bei 25°C flüssig ist.

5. Kosmetische Zusammensetzung gemäß Anspruch 3 oder 4, wobei mindestens ein Teil des Ölmittels (b) eine Siliconverbindung außer den durch Formel (1) verkörperten Siliconverbindungen ist.

6. Kosmetische Zusammensetzung gemäß einem beliebigen der Ansprüche 3 bis 5, wobei mindestens ein Teil des Ölmittels (b) eine Siliconverbindung ist, ausgewählt aus der Gruppe, bestehend aus linearen Organopolysiloxanen, verkörpert durch die folgende allgemeine Formel (2), cyclischen Organopolysiloxanen, verkörpert durch die allgemeine Formel (3), und verzweigten Organopolysiloxanen, verkörpert durch die allgemeine Formel (4): 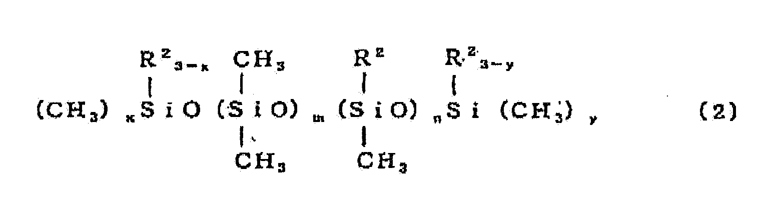 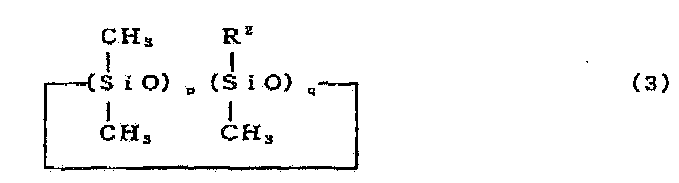
(CH₃)₄₋ᵣSi{OSi(CH₃)₃}ᵣ (4)
worin R² unabhängig voneinander eine Gruppe verkörpert, ausgewählt aus der Gruppe, bestehend aus einem Wassenstoffatom, einer Hydroxylgruppe, einwertigen unsubstituierten oder fluorsubstituierten Alkylgruppen, Arylgruppen, aminosubstituierten Alkylgruppen, Alkoxygruppen mit 2 bis 20 Kohlenstoffatomen, und einer Gruppe, verkörpert durch die allgemeine Formel: (CH₃)₃SiO{ (CH₃)₂SiO}s Si (CH₃)₂CH₂CH₂-, m eine ganze Zahl von 0 bis 1.000 ist, n eine ganze Zahl von 0 bis 1.000 ist, m + n eine ganze Zahl von 1 bis 2.000 ist, x und y jeweils 0, 1, 2 oder 3 sind, p und q jeweils eine ganze Zahl von 0 bis 8 sind, wobei 3 ≤ p + q ≤8, r eine ganze Zahl von 1 bis 4 ist und s eine ganze Zahl von 0 bis 500 ist.

7. Kosmetische Zusammensetzung gemäß Anspruch 6, wobei mindestens ein Teil des Ölmittels ausgewählt ist aus der Gruppe, bestehend aus den linearen Organopolysiloxanen der allgemeinen Formel (2), wobei mindestens ein Teil von R² eine aminosubstituierte oder fluorsubstituierte Alkylgruppe mit 2 bis 20 Kohlenstoffatomen ist, einem cyclischen Organopolysiloxan, verkörpert durch die allgemeine Formel (3), enthaltend eine - {(CF₃CH₂CH₂) (CH₃) SiO}_{q}-Einheit, Perfluorpolyether, Perfluordecalin und Perfluoroctan.

8. Kosmetische Zusammensetzung gemäß einem beliebigen der Ansprüche 1 bis 7, die des Weiteren eine Verbindung mit einer alkoholischen Hydroxylgruppe (c) außer höheren Alkoholen umfasst.

9. Kosmetische Zusammensetzung gemäß Anspruch 8, wobei die Verbindung (c) ein wasserlöslicher ein- oder mehrwertiger Alkohol mit 2 bis 10 Kohlenstoffatomen ist:

10. Kosmetische Zusammensetzung gemäß einem beliebigen der Ansprüche 1 bis 9, die des Weiteren ein wasserlösliches Polymer und/oder ein wasserquellbares Polymer (d) umfasst.

11. Kosmetische Zusammensetzung gemäß einem beliebigen der Ansprüche 1 bis 10, die des Weiteren ein Pulver bzw. einen Puder und/oder ein Färbemittel umfasst.

12. Kosmetische Zusammensetzung gemäß Anspruch 11, wobei mindestens ein Teil des Pulvers bzw. Puders und/oder Färbemittels ausgewählt ist aus der Gruppe, bestehend aus sphärischem Siliconelastomer-Pulver, sphärischem Polymethylsilsesquioxan-Pulver, sphärischem Siliconelastomer-Pulver, dessen Oberfläche mit Polymethylsilsesquioxan beschichtet ist, Polyethylenpulver, Polypropylenpulver, Polytetrafluorethylenpulver und Polyurethanpulver.

13. Kosmetische Zusammensetzung gemäß einem beliebigen der Ansprüche 1 bis 12, die des Weiteren ein Tensid (g) umfasst.

14. Kosmetische Zusammensetzung gemäß einem beliebigen der Ansprüche 1 bis 13, das des Weiteren ein vernetztes Organopolysiloxan (h) umfasst.

15. Kosmetische Zusammensetzung gemäß Anspruch 14, wobei das vernetzte Organopolysiloxan (h) in gequollener Form vorliegt, wobei es mit einer größeren gewichtsmäßigen Menge als der Menge des Organopolysiloxans (h) an Silicon mit einer Viskosität von 0,65 mm²/sec bis 100 mm²/sec gequollen wird.

16. Kosmetische Zusammensetzung gemäß Anspruch 14 oder 15, wobei das vernetzte Organopolysiloxan (h) durch Umsetzen eines Organopolysiloxans mit 2 oder mehr reaktiven Vinylstellen mit einem Organohydrogenpolysiloxan mit einem Wasserstoffatom, das an ein Siliciumatom gebunden ist, erhalten wird.

17. Kosmetische Zusammensetzung gemäß einem beliebigen der Ansprüche 14 bis 16, wobei das vernetzte Organopolysiloxan (h) mindestens einen Rest besitzt, ausgewählt aus der Gruppe, bestehend aus Polyoxyalkylenresten, Alkylresten, Arylresten, Polyglycerinresten und Fluoralkylresten.

18. Kosmetische Zusammensetzung gemäß einem beliebigen der Ansprüche 1 bis 17, die des Weiteren ein Siliconharz (i) umfasst, das bei 25 Grad C gummiartig oder fest ist.

19. Kosmetische Zusammensetzung gemäß Anspruch 18, wobei das Siliconharz (i) mindestens eine Netzwerksiliconverbindung, verkörpert durch MQ, MT, MDQ, ,MDT, MTQ, MDTQ, TD, TQ oder TDQ, darstellt.

20. Kosmetische Zusammensetzung gemäß Anspruch 18 oder 19, wobei das Siliconharz (i) eine Netzwerksiliconverbindung mit mindestens einem Rest, ausgewählt aus der Gruppe, bestehend aus Pyrrolidonresten, langkettigen Alkylresten, Polyoxyalkylenresten, Fluoralkylresten und Aminoresten ist.

21. Kosmetische Zusammensetzung gemäß einem beliebigen der Ansprüche 18 bis 20, wobei das Siliconharz (i) ein Acrylsiliconharz ist.

22. Kosmetische Zusammensetzung nach einem beliebigen der Ansprüche 1 bis 21, das des Weiteren eine vor UV schützende Komponente (j) umfasst.

23. Kosmetische Zusammensetzung nach einem beliebigen der Ansprüche 1 bis 22, wobei die kosmetische Zusammensetzung ein Hautpflegekosmetikum, ein Haarpflegekosmetikum, ein Antitranspirationsmittel, ein Make-up-Kosmetikum oder ein vor UV schützendes Kosmetikum ist.

24. Kosmetische Zusammensetzung gemäß einem beliebigen der Ansprüche 1 bis 23, wobei die kosmetische Zusammensetzung in Form einer Flüssigkeit, Emulsion, Creme, eines Feststoffs, einer Paste, eines Gels, eines Puders, Mehrfachschichten, eines Schaums ("^{,}mousse") oder eines Sprays vorliegt.

## Revendications

1. Composition cosmétique comprenant
de 5 à 50 % en masse d'un composé de silicone (a) représenté par la formule générale (1) suivante,
{(CH₃)₃SiO}₃SiR¹ (1)
dans laquelle R¹ représente un groupe alkyle monovalent ayant de 2 à 20 atomes de carbone,
de 0,1 à 20 % en masse d'au moins un tensioactif non ionique choisi parmi un organopolysiloxane polyoxyalkylène-modifié, un organopolysiloxane polyglycérine-modifié et un organopolysiloxane polyoxyalkylène/alkyle co-modifié, et de l'eau.

2. Composition cosmétique selon la revendication 1, dans laquelle le tensioactif non ionique est un organopolysiloxane polyoxyalkylène-modifié.

3. Composition cosmétique selon la revendication 1 ou 2, laquelle comprend de plus un agent d'huile (b).

4. Composition cosmétique selon la revendication 3, dans laquelle au moins une partie dudit agent d'huile (b) est liquide à 25°C.

5. Composition cosmétique selon la revendication 3 ou 4, dans laquelle au moins une partie de l'agent d'huile (b) est un composé de silicone différent des composés de silicone représentés par la formule (1).

6. Composition cosmétique selon l'une quelconque des revendications 3 à 5, dans laquelle au moins une partie de l'agent d'huile (b) est un composé de silicone choisi parmi des organopolysiloxanes linéaires représentés par la formule générale (2) suivante, des organopolysiloxanes cycliques représentés par la formule générale (3) et des organopolysiloxanes ramifiés représentés par la formule générale (4) : 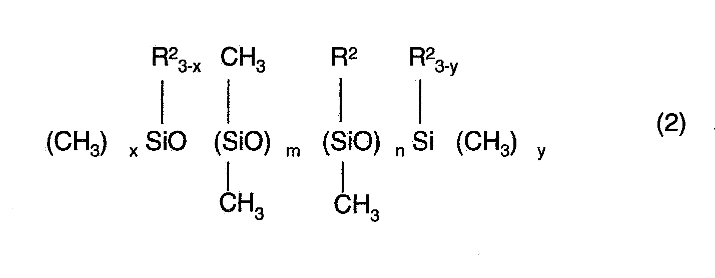 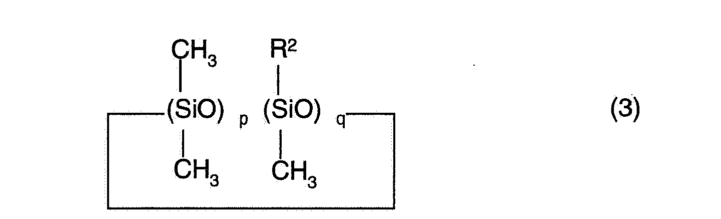
(CH₃)₄₋ᵣSi{OSi(CH₃)₃}ᵣ (4)
dans lesquelles R² représente indépendamment un groupe choisi parmi un atome d'hydrogène, un groupe hydroxyle, des groupes alkyle substitués par du fluor ou non substitués monovalents, des groupes aryle, des groupes alkyle amino-substitués, des groupes alcoxy ayant de 2 à 20 atomes de carbone et un groupe représenté par la formule générale : (CH₃)₃SiO{(CH₃)₂SiO}s Si(CH₃)₂CH₂CH₂-, m est un nombre entier de 0 à 1000, n est un nombre entier de 0 à 1000, m + n est un nombre entier de 1 à 2000, x et y sont chacun égaux à 0, 1, 2 ou 3, p et q sont chacun un nombre entier de 0 à 8 avec 3 ≤ p + q ≤ 8, r est un nombre entier de 1 à 4, et s est un nombre entier de 0 à 500.

7. Composition cosmétique selon la revendication 6, dans laquelle au moins une partie de l'agent d'huile est choisie parmi des organopolysiloxanes linéaires de la formule générale (2), dans laquelle au moins une partie de R² est un groupe alkyle amino-substitué ou substitué par du fluor présentant 2-20 atomes de carbone, un organopolysiloxane cyclique représenté par la formule générale (3) contenant une unité -{(CF₃CH₂CH₂)(CH₃)SiO}_{q-}, perfluoropolyéther, perfluorodécaline et perfluorooctane.

8. Composition cosmétique selon l'une quelconque des revendications 1 à 7, laquelle comprend de plus un composé présentant un groupe hydroxyle alcoolique (c) différent des alcools supérieurs.

9. Composition cosmétique selon la revendication 8, dans laquelle le composé (c) est un alcool mono- ou polyvalent soluble, dans l'eau ayant de 2 à 10 atomes de carbone.

10. Composition cosmétique selon l'une quelconque des revendications 1 à 9, laquelle comprend de plus un polymère soluble dans l'eau et/ou un polymère gonflant dans l'eau (d).

11. Composition cosmétique selon l'une quelconque des revendications 1 à 10, laquelle comprend de plus une poudre et/ou un agent colorant.

12. Composition cosmétique selon la revendication 11, dans laquelle au moins une partie de la poudre et/ou d'un agent colorant est choisie parmi une poudre d'élastomère de silicone sphérique, une poudre de polyméthylsilsesquioxane sphérique, une poudre d'élastomère de silicone sphérique avec sa surface revêtue de polyméthylsilsesquioxane, une poudre de polyéthylène, une poudre de polypropylène, une poudre de polytétrafluoroéthylène et une poudre de polyuréthanne.

13. Composition cosmétique selon l'une quelconque des revendications 1 à 12, laquelle comprend de plus un tensioactif (g).

14. Composition cosmétique selon l'une quelconque des revendications 1 à 13, laquelle comprend de plus un organopolysiloxane réticulé (h).

15. Composition cosmétique selon la revendication 14, dans laquelle l'organopolysiloxane réticulé (h) est dans une forme gonflée où il est gonflé avec une quantité en poids de silicone présentant une viscosité de 0,65 mm²/s à 100 mm²/s supérieure à la quantité de l'organopolysiloxane (h).

16. Composition cosmétique selon la revendication 14 ou 15, dans laquelle l'organopolysiloxane réticulé (h) est un organopolysiloxane obtenu par réaction d'un organopolysiloxane présentant deux ou plusieurs sites réactifs vinyliques avec un organohydrogénopolysiloxane présentant un atome d'hydrogène qui est lié à un atome de silicium.

17. Composé cosmétique selon l'une quelconque des revendications 14 à 16, dans laquelle l'organopolysiloxane réticulé (h) présente au moins un résidu choisi parmi des résidus de polyoxyalkylène, des résidus d'alkyle, des résidus d'aryle, des résidus de polyglycérine et des résidus de fluoroalkyle.

18. Composition cosmétique selon l'une quelconque des revendications 1 à 17, laquelle comprend de plus une résine de silicone (i) qui est dans la forme de gomme ou solide à 25°C.

19. Composition cosmétique selon la revendication 18, dans laquelle la résine de silicone (i) est au moins un composé de silicone en réseau représenté par MQ, MT, MDQ, MDT, MTQ, MDTQ, TD, TQ ou TDQ.

20. Composition cosmétique selon la revendication 18 ou 19, dans laquelle la résine de silicone (i) est un composé de silicone en réseau présentant au moins un résidu choisi parmi des résidus de pyrrolidone, des résidus alkyle à chaîne longue, des résidus de polyoxyalkylène, des résidus de fluoroalkyle et un résidu amino.

21. Composition cosmétique selon l'une quelconque des revendications 18 à 20, dans laquelle la résine de silicone (i) est une résine de silicone acrylique.

22. Composition cosmétique selon l'une quelconque des revendications 1 à 21, laquelle comprend de plus un constituant protecteur contre les UV (j).

23. Composition cosmétique selon l'une quelconque des revendications 1 à 22, dans laquelle la composition cosmétique est un cosmétique pour le soin de la peau, un cosmétique pour le soin capillaire, un antiperspirant, un cosmétique de maquillage ou un cosmétique de protection contre les UV.

24. Composition cosmétique selon l'une quelconque des revendications 1 à 23, dans laquelle la composition cosmétique est dans une forme de liquide, d'émulsion, de crème, de solide, de pâte, de gel, de poudre, de multicouches, de mousse ou de pulvérisation.
